(19) Europäisches Patentamt
European Patent Office
Office européen des brevets

(11) EP 2 618 808 B1

(12) # FASCICULE DE BREVET EUROPEEN

(45) Date de publication et mention de la délivrance du brevet:
**21.09.2016 Bulletin 2016/38**

(21) Numéro de dépôt: **11773870.8**

(22) Date de dépôt: **23.09.2011**

(51) Int Cl.:
***G01N 33/68*** $^{(2006.01)}$ ***C07K 14/47*** $^{(2006.01)}$
***A61K 8/64*** $^{(2006.01)}$ ***A61Q 19/08*** $^{(2006.01)}$
***A61K 8/60*** $^{(2006.01)}$

(86) Numéro de dépôt international:
**PCT/IB2011/054190**

(87) Numéro de publication internationale:
**WO 2012/038929 (29.03.2012 Gazette 2012/13)**

(54) **UTILISATION COSMÉTIQUE DE LA DERMCIDINE OU FRAGMENTS DE CELLE-CI**

KOSMETISCHE VERWENDUNG VON DERMICIDIN UND FRAGMENTE DAVON

COSMETIC USE OF DERMICIDIN AND FRAGMENTS THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.10.2010 US 344827 P**
**19.10.2010 US 344826 P**
**24.09.2010 FR 1057699**
**24.09.2010 FR 1057690**

(43) Date de publication de la demande:
**31.07.2013 Bulletin 2013/31**

(73) Titulaire: **L'Oréal**
**75008 Paris (FR)**

(72) Inventeurs:
- **DELATTRE, Caroline**
  **F-95470 Vemars (FR)**
- **GUENICHE, Audrey**
  **F-92500 Rueil-malmaison (FR)**
- **CASTIEL, Isabelle**
  **F-06200 Nice (FR)**
- **BERNARD, Dominique**
  **F-92170 Vanves (FR)**

(74) Mandataire: **Nony**
**11 rue Saint-Georges**
**75009 Paris (FR)**

(56) Documents cités:
**EP-A1- 2 206 493** **FR-A1- 2 924 946**
**US-A- 5 834 192** **US-A1- 2008 020 976**

- **GUENICHE A ET AL: "Reactive Skin: Clinical efficacy of a probiotic lysate is associated with specific changes in stratum corneum protein pattern", JOURNAL OF INVESTIGATIVE DERMATOLOGY, vol. 129, no. Suppl. 2, septembre 2009 (2009-09), page S29, XP002628494, & 39TH ANNUAL MEETING OF THE EUROPEAN-SOCIETY-FOR-DERMATOLOGICAL -RESEA RCH; BUDAPEST, HUNGARY; SEPTEMBER 09 -12, 2009 ISSN: 0022-202X**
- **GUENICHE A ET AL: "Probiotic lysate applied to skin: in vitro evidences of beneficial effects of Bifidobacterium longum sp on sensitive skin and aging", JOURNAL OF INVESTIGATIVE DERMATOLOGY, vol. 129, no. Suppl. 2, septembre 2009 (2009-09), page S64, XP002628492, & 39TH ANNUAL MEETING OF THE EUROPEAN-SOCIETY-FOR-DERMATOLOGICAL -RESEA RCH; BUDAPEST, HUNGARY; SEPTEMBER 09 -12, 2009 ISSN: 0022-202X**
- **GUÉNICHE AUDREY ET AL: "Bifidobacterium longum lysate, a new ingredient for reactive skin.", EXPERIMENTAL DERMATOLOGY AUG 2010 LNKD- PUBMED:19624730, vol. 19, no. 8, août 2010 (2010-08), pages E1-E8, XP002628493, ISSN: 1600-0625**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 618 808 B1

## Description

Le présent brevet se rapporte au domaine des biomarqueurs de la peau, et plus particulièrement de la peau âgée, associée ou non à une sécheresse cutanée, ainsi qu'à leur mise en oeuvre à titre de cibles ou d'agents actifs cosmétiques.

L'épiderme, partie superficielle de la peau, est un tissu dont les cellules sont jointives et solidaires les unes des autres et reposent sur une membrane basale. Il forme un revêtement externe comprenant des glandes sébacées ou sudoripares et les follicules pileux.

Plus précisément, l'épiderme est une structure dont l'homéostasie résulte de la mise en oeuvre d'un ensemble finement régulé de signaux intracellulaires et extracellulaires agissant à toutes les étapes de la prolifération, de la migration, de la différenciation cellulaire, ainsi que de la synthèse des différents composants de la matrice extracellulaire. Ces signaux peuvent, notamment, résulter de l'action de facteurs produits par les kératinocytes.

L'épiderme est conventionnellement divisé en une couche basale de kératinocytes contenant, notamment, des cellules souches cutanées et constituant la couche germinative de l'épiderme, une couche dite épineuse constituée de plusieurs couches de cellules polyhédriques disposées sur la couche basale, une couche dite granuleuse comprenant une à trois couches dites de cellules aplaties contenant des inclusions cytoplasmiques distinctes, les grains de kératohyaline, et enfin, un ensemble de couches supérieures, appelé couche cornée, ou *stratum corneum,* constituée de kératinocytes au stade terminal de leur différenciation, appelés cornéocytes.

Le *stratum corneum,* la partie la plus externe de la peau qui assure la fonction barrière entre l'organisme et l'environnement, et la tige pilaire, partie émergente du follicule pileux qui constitue la chevelure, représentent tous deux l'aboutissement du processus de différenciation des kératinocytes. La différenciation épidermique suit un processus de maturation dans lequel des kératinocytes de la couche basale se différencient et migrent pour aboutir à la formation des cornéocytes, cellules mortes totalement kératinisées. Cette différenciation est la résultante de phénomènes parfaitement coordonnés qui vont conduire au maintien de l'homéostasie de l'épiderme, et conférer à la peau un aspect sain, jeune, lumineux et lisse.

Lors du vieillissement, de nombreuses altérations physiologiques de la peau, résultant d'un dysfonctionnement de l'homéostasie de l'épiderme, et notamment d'un dysfonctionnement de la différenciation épithéliale des kératinocytes et/ou de la synthèse des protéoglycanes, se produisent.

Les altérations de l'homéostasie de l'épiderme se produisant lors du vieillissement se traduisent principalement par une diminution de la différenciation des kératinocytes entraînant un déficit de la matrice protéique des cellules cornées, par une augmentation des métalloprotéases, et de leur activité de dégradation de la matrice extracellulaire, ainsi que par une diminution de la synthèse des différents glycosaminoglycannes.

Lors du vieillissement de la peau, ces altérations se traduisent, généralement, par l'apparition d'un microrelief de la peau plus marqué, voire de ridules, et au final la survenue de rides profondes, une perte de l'élasticité, un toucher rugueux, et une sècheresse cutanée. Sur le plan histologique, un aplatissement de la jonction dermo-épidermique et une diminution de l'épaisseur du derme et de l'épiderme sont observés.

Des troubles de l'hydratation cutanée, et notamment une sècheresse cutanée, peuvent être en outre souvent observés avec l'âge.

Qui plus est, de nombreux facteurs extérieurs peuvent en outre renforcer l'assèchement de la peau ou aggraver cet état. Parmi ces facteurs, on peut citer les conditions climatiques telles que le froid ou le vent, les rayons solaires, l'exposition à certains agents chimiques ou thérapeutiques.

Sur le plan physiologique, une peau sèche est souvent associée à une baisse du taux d'hydratation cutanée ainsi qu'à une modification du processus de maturation du *stratum corneum* ayant pour signe le plus visible l'apparition de squames à la surface cutanée. Sur le plan sensoriel, une peau sèche peut être caractérisée par une sensation de tiraillement et/ou de tension cutanée.

Le contenu en collagène et en glycosaminoglycannes de la peau est également diminué et la fonction barrière de la peau âgée peut être altérée.

De nombreux facteurs épidermiques, dont l'expression, l'activité biologique ou la maturation sont altérées, diminuées ou augmentées, sont connus pour être impliqués, directement ou indirectement, dans la survenue et la manifestation de la peau âgée ou des différents signes du vieillissement cutané associés, comme la sécheresse cutanée.

Ces facteurs peuvent être utilisés à titre de bio-marqueurs de la peau, comme cibles de criblage, voire comme actifs cosmétiques.

Par exemple, on connait du document FR 2 924 946 l'utilisation cosmétique et/ou thérapeutique de la céramidase acide, de polypeptides dérivés de cette protéine ou d'analogues de ceux-ci, d'une séquence nucléique codant un tel polypeptide ou d'un agent modulateur de l'expression ou de l'activité , biologique ou biochimique, d'un tel polypeptide, pour prévenir et/ou traiter les signes du vieillissement cutané, ainsi qu'à titre de marqueur pour l'évaluation d'un état d'un épithélium.

Toutefois, il demeure encore de nombreuses inconnues quant au mécanisme intime et quant à l'ensemble des facteurs impliqués dans le vieillissement de la peau, associé ou non à une déshydratation.

Ainsi, il demeure un besoin d'identifier de nouveaux biomarqueurs de la peau, et notamment aptes à caractériser une peau âgée ou des signes du vieillissement cutané, en particulier une peau âgée et sèche ou des signes de sécheresse cutanée associés à une peau âgée.

Il existe également un besoin de disposer de nouveaux biomarqueurs permettant de caractériser un état de la peau, et en particulier une peau âgée ou des signes du vieillissement cutané, en particulier une peau âgée et sèche ou des signes de sécheresse cutanée associés à une peau âgée.

Il existe encore un besoin de disposer de nouveaux outils de criblage d'agents actifs ou de traitements physiques convenant au soin de la peau, et plus particulièrement utiles pour prévenir et/ou traiter une peau âgée ou des signes du vieillissement cutané, en particulier une peau âgée et sèche ou des signes de sécheresse cutanée associés à une peau âgée.

Il existe encore un besoin de disposer de nouveaux actifs ou de nouveaux traitements pour la prévention et/ou le traitement d'une peau âgée ou des signes du vieillissement cutané, en particulier une peau âgée et sèche ou des signes de sécheresse cutanée associés à une peau âgée.

Il existe encore un besoin de disposer de nouvelles cibles cosmétiques pour le soin de la peau, et en particulier pour la prévention et/ou le traitement d'une peau âgée ou des signes du vieillissement cutané, associés ou non à une sécheresse cutanée.

La présente invention a pour objet de satisfaire à ces besoins.

La présente invention concerne, selon un de ses premiers objets, l'utilisation (i) d'au moins une séquence d'acides aminés codés par une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10 ou (ii) de ladite séquence d'acides nucléiques, à titre de biomarqueur d'un état de la peau âgée et/ou des signes cutanés du vieillissement, associé(e)(s) ou non à une sécheresse cutanée.

Dans le présent brevet, est également décrite l'utilisation (i) d'au moins une séquence d'acides aminés codés par une séquence d'acides nucléiques représentés par SEQ ID NO: 1, d'un analogue ou d'un fragment de ladite séquence d'acides aminés, ou (ii) de ladite séquence d'acides nucléiques, à titre de biomarqueur d'un état d'hydratation de la peau, et en particulier de la peau sèche et/ou des signes de sècheresse cutanée.

Au sens de l'invention, on entend par « biomarqueur », une molécule ou l'activité d'une molécule, dont la présence, la teneur ou le degré d'activité est caractéristique d'un processus biologique, physiologique ou pathologique, ou de l'impact ou de l'effet induit par l'administration d'un agent actif ou d'un traitement physique sur un tel processus.

Au sens de l'invention, on entend désigner par « peau » l'ensemble de l'épiderme du corps humain, y compris le cuir chevelu et les lèvres. Plus particulièrement, la peau considérée dans la présente invention est de préférence les lèvres, la peau du visage, du décolleté, des bras ou des jambes, et de manière préférée, la peau du visage et/ou du décolleté.

De manière inattendue, au cours d'une étude de protéomique différentielle par la technologie « iTRAQ », les inventeurs ont observé à partir de protéines extraites de strippings vernis de populations de classes d'âge différentes que la Dermcidine (ou DCD) se révélait être un biomarqueur sensible et spécifique de la peau âgée.

Par ailleurs, il est à noter que la séquence d'acide nucléique SEQ ID NO :1, codant la séquence d'acide aminé SEQ ID NO :11, a également été décrite comme étant le polynucleotide hcap (pour « human cachexia-associated protein »), isolé à partir d'une librairie de cancer du sein, tel que décrit dans le brevet US 5,834,192.

Plus précisément, les inventeurs ont observé que le taux d'expression de la Dermcidine, et plus particulièrement de peptides issus de la Dermcidine et identifiés par les séquences SEQ ID NO: 17 et SEQ ID NO: 18 étaient systématiquement diminués dans les peaux âgées par rapport aux peaux jeunes.

En outre, de manière inattendue, au cours d'une étude de protéomique comparative par Western Blot, les inventeurs ont observé à partir de protéines extraites de strippings vernis de populations de classes d'âge différentes présentant différents degrés d'hydratation cutanée que la Dermcidine (ou DCD) se révélait être un biomarqueur sensible et spécifique de la peau sèche.Ainsi, les inventeurs ont observé que le taux d'expression de la Dermcidine, et plus particulièrement d'un peptide identifié par la séquence SEQ ID NO: 16 était systématiquement diminué dans les peaux sèches par rapport aux peaux normalement hydratées. A la connaissance des inventeurs, cette protéine antimicrobienne de la surface cutanée n'a jamais été classée comme variant au cours du vieillissement ou lors de la déshydratation de la peau et encore moins comme l'un des biomarqueurs les plus pertinents ressortant de ce type d'études.

Il est connu que la sudation diminue avec l'âge (Anderson and Kenney 1987 ; Kenney and Fowler 1988) et que la Dermcidine est une protéine sudoripare (Schittek, Hipfel *et al.,* 2001). En revanche, les données expérimentales obtenues par les inventeurs montrent de façon inattendue qu'il existe un déficit spécifique d'expression et/ou de maturation de la DCD au cours du vieillissement cutané, car celle-ci est sous-représentée dans les échantillons cutanés, même par rapport à d'autres protéines d'origine sudoripare. Par ailleurs, il peut être souligné que la sécheresse cutanée est un phénomène complexe qui n'est pas systématiquement associé à un déficit sudoripare.

Selon encore un autre de ses objets, la présente invention concerne l'utilisation (i) d'au moins une séquence d'acides aminés représentée par une séquence choisie parmi SEQ ID NO: 11, SEQ ID NO : 12, SEQ ID NO : 13 et SEQ ID NO : 16, ou (ii) d'au moins une séquence d'acides nucléiques représentée par une séquence choisie parmi SEQ ID NO: 1, SEQ ID NO : 2, SEQ ID NO : 3 et SEQ ID NO : 6, pour cribler des agents actifs ou des traitements physiques pour le soin de la peau âgée et/ou la prévention et/ou le traitement des signes cutanés du vieillissement, associé(e)s ou non à une sécheresse cutanée, susceptibles de moduler l'activité, l'expression ou la maturation de ladite séquence d'acides aminés ou de ladite séquence d'acides nucléiques.

Les agents actifs ou les traitements physiques criblés peuvent être plus particulièrement destinés à prévenir et/ou traiter la peau âgée et/ou les signes cutanés du vieillissement, associé(e)(s) ou non à une sécheresse cutanée.

Dans le présent brevet, est également décrit une utilisation (i) d'au moins une séquence d'acides aminés, ou (ii) d'une séquence d'acides nucléiques, pour cribler des agents actifs ou des traitements physiques susceptibles de moduler l'activité, l'expression ou la maturation de ladite séquence d'acides aminés ou de ladite séquence d'acides nucléiques et destinés à favoriser et/ou renforcer l'hydratation de la peau.

De manière préférée, de tels agents actifs ou traitements physiques peuvent être destinés à prévenir et/ou traiter la peau sèche et/ou les signes de sècheresse cutanée.

Au sens de l'invention on entend par « expression » à l'égard d'une séquence d'acides aminés, par exemple une protéine ou un peptide, ou d'une séquence d'acides nucléiques, par exemple un ARNm, sa teneur ou la variation de sa teneur par rapport à une référence.

Au sens de l'invention on entend par « maturation » à l'égard d'une séquence d'acides aminés, par exemple une protéine ou un peptide, ou d'une séquence d'acides nucléiques, par exemple un ARNm, les modifications qui suivent leur synthèse dans un environnement cellulaire. Par exemple, dans le cas d'une séquence d'acides aminés, on entend par « maturation », les modifications post-traductionnelles, telle la glycosylation ou la farnésylation de certains acides aminés, ou les étapes protéolytiques conduisant à l'élimination de séquences dites « signal » ou « sécrétoire » ou à la libération de séquences présentant des propriétés biologiques particulières. Dans le cas d'une séquence d'acides nucléiques, on entend par « maturation », par exemple, l'épissage alternatif d'un ARNm.

Au sens de l'invention on entend par « activité », à l'égard d'une séquence d'acides aminés, par exemple une protéine ou un peptide, l'activité biologique de la séquence d'acides aminés, le cas échéant après maturation, telle qu'une activité enzymatique, une activité d'agoniste ou d'antagoniste vis-à-vis d'un récepteur, ou d'activateur ou d'inhibiteur d'une enzyme, une activité dite de « structure », ou une activité antimicrobienne.

Au sens de l'invention on entend par « activité », à l'égard d'une séquence d'acides nucléiques, par exemple un ARNm, sa traduction.

Selon un autre de ses objets, la présente invention concerne l'utilisation (i) d'au moins une séquence d'acides aminés représentée par une séquence choisie parmi SEQ ID NO: 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, ou (ii) d'au moins une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, pour caractériser l'efficacité d'un traitement cosmétique de la peau âgée et/ou les signes cutanés du vieillissement, associé(e)(s) ou non à une sécheresse cutanée.

Dans le présent brevet, est encore décrit une utilisation (i) d'au moins une séquence d'acides aminés, ou (ii) d'au moins une séquence d'acides nucléiques, pour caractériser l'efficacité d'un traitement cosmétique de la peau sèche et/ou des signes de sécheresse cutanée.

Selon encore un autre de ses objets, la présente invention concerne l'utilisation cosmétique d'une quantité efficace (i) d'au moins une séquence d'acides aminés représentée par une séquence choisie parmi SEQ ID NO: 11, SEQ ID NO : 12, SEQ ID NO : 13 et SEQ ID NO : 16, ou (ii) d'au moins une séquence d'acides nucléiques représentée par une séquence choisie parmi SEQ ID NO: 1, SEQ ID NO : 2, SEQ ID NO : 3 et SEQ ID NO : 6, à titre d'agent actif pour prévenir et/ou traiter la peau âgée et/ou les signes cutanés du vieillissement, associé(e)(s) ou non à une sécheresse cutanée.

Dans le présent brevet, est également décrit une utilisation cosmétique d'une quantité efficace (i) d'au moins une séquence d'acides aminés, ou (ii) d'au moins une séquence d'acides nucléiques, ou (iii) d'au moins un agent modulateur de l'activité, de l'expression ou de la maturation de ladite séquence d'acides aminés ou de ladite séquence d'acides nucléiques, à titre d'agent actif pour favoriser et/ou renforcer l'hydratation de la peau.

De manière préférée, une telle utilisation peut être dédiée à prévenir et/ou traiter la peau sèche et/ou les signes de sècheresse cutanée.

Au sens de la présente invention, on entend par « quantité efficace » d'un composé de l'invention, une quantité suffisante et nécessaire de ce composé pour obtenir un effet recherché, et plus particulièrement un effet cosmétique ou de soin à l'égard de la peau âgée et/ou des signes cutanés du vieillissement, associé(e)(s) ou non à une sécheresse cutanée. Egalement, une « quantité efficace » d'un composé peut être une quantité suffisante et nécessaire de ce composé pour obtenir un effet cosmétique ou de soin à l'égard de l'hydratation de la peau, et en particulier de la peau sèche et/ou des signes de sècheresse cutanée.

Au sens de l'invention, on entend par « prévenir », le fait de réduire le risque de survenue ou de ralentir la survenue d'un phénomène donné, à savoir dans la présente invention, la peau âgée et/ou les signes du vieillissement cutané, associé(e)(s) ou non à une sécheresse cutanée. Egalement, au sens du brevet, on entend par « prévenir », le fait de réduire le risque de survenue ou de ralentir la survenue d'une peau sèche et/ou des signes de sècheresse cutanée.

Selon encore un autre de ses objets, la présente invention concerne l'utilisation d'une quantité efficace (i) d'au moins une séquence d'acides aminés représentée par une séquence choisie parmi SEQ ID NO: 11, SEQ ID NO : 12, SEQ ID NO : 13 et SEQ ID NO : 16, ou (ii) d'au moins une séquence d'acides nucléiques représentée par une séquence choisie parmi SEQ ID NO: 1, SEQ ID NO : 2, SEQ ID NO : 3 et SEQ ID NO : 6, pour préparer un modèle cellulaire épithéliale pluristratifié, de préférence un modèle de peau reconstruite, mimant une peau âgée, éventuellement associée à une sécheresse cutanée.

Selon encore un autre de ses objets, la présente invention concerne un procédé *in vitro* ou *ex vivo* pour caractériser un état de la peau âgée et/ou des signes cutanés du vieillissement, associé(e)(s) ou non à une sécheresse cutanée, comprenant au moins les étapes consistant à :

a) effectuer, dans un échantillon isolé d'une peau, une mesure qualitative ou quantitative de l'expression, de la maturation ou de l'activité d'au moins une séquence d'acides aminés représentée par une séquence choisie parmi SEQ ID NO: 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20 ou d'au moins une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, et
b) comparer ladite mesure effectuée à l'étape a) à une mesure de référence.

Selon la différence observée entre la mesure obtenue et la mesure de référence, la peau sera qualifiée de peau normalement jeune ou de peau âgée, voire âgée et sèche.

Selon un mode de réalisation préféré, un état de la peau considéré dans l'invention est une peau âgée choisie parmi des peaux ayant subi un vieillissement chronologique et/ou un vieillissement photo-induit.

Dans le présent brevet, est également décrit un procédé pour caractériser un état d'hydratation de la peau, et en particulier une peau sèche et/ou des signes cutanés de sècheresse, comprenant au moins les étapes consistant à :

a) effectuer, dans un échantillon isolé d'une peau, une mesure qualitative ou quantitative de l'expression, de la maturation ou de l'activité de ladite séquence d'acides aminés ou de ladite séquence d'acides nucléiques, et
b) comparer ladite mesure effectuée à l'étape a) à une mesure de référence.

Selon la différence observée entre la mesure obtenue et la mesure de référence, la peau sera qualifiée de peau normalement hydratée ou de peau sèche.

Selon encore un autre de ses objets, le présent brevet décrit un procédé de criblage d'agents actifs ou de traitements physiques susceptibles de moduler l'activité, l'expression ou la maturation d'une séquence d'acides aminés de l'invention ou d'une séquence d'acides nucléiques de l'invention, comprenant au moins les étapes consistant à :

a) disposer ladite séquence d'acides aminés ou ladite séquence d'acides nucléiques dans des conditions propices à l'activité, l'expression ou la maturation desdites séquences,
b) mettre en contact ladite séquence d'acides aminés ou ladite séquence d'acides nucléiques avec au moins un agent actif à tester, ou exposer ladite séquence d'acides aminés ou ladite séquence d'acides nucléiques avec un traitement physique à tester,
c) effectuer une mesure qualitative ou quantitative de l'expression, de la maturation ou de l'activité de ladite séquence d'acides aminés ou de ladite séquence d'acides nucléiques, et
d) comparer ladite mesure à une mesure de référence.

Selon la différence observée entre la mesure obtenue et la mesure de référence, le composé ou le traitement physique criblé seront caractérisés ou non comme étant utiles pour prévenir et/ou du traiter la peau âgée et/ou les signes cutanés du vieillissement, associé(e)(s) ou non à une sécheresse cutanée.

Selon un mode de réalisation , le ou les agents actifs ou le ou les traitements physiques criblés peuvent être plus particulièrement dédiés à prévenir et/ou traiter la peau âgée et/ou les signes cutanés du vieillissement, associé(e)(s) ou non à une sécheresse cutanée.

Dans le présent brevet, est également décrit un procédé de criblage d'agents actifs ou de traitements physiques susceptibles de moduler l'activité, l'expression ou la maturation d'une séquence d'acides aminés ou d'une séquence d'acides nucléiques et destinés à favoriser et/ou renforcer l'hydratation de la peau sèche, comprenant au moins les étapes consistant à :

a) disposer ladite séquence d'acides aminés ou ladite séquence d'acides nucléiques dans des conditions propices à l'activité, l'expression ou la maturation desdites séquences,
b) mettre en contact ladite séquence d'acides aminés ou ladite séquence d'acides nucléiques avec au moins un agent actif à tester, ou exposer ladite séquence d'acides aminés ou ladite séquence d'acides nucléiques avec un traitement physique à tester,
c) effectuer une mesure qualitative ou quantitative de l'expression, de la maturation ou de l'activité de ladite séquence d'acides aminés ou de ladite séquence d'acides nucléiques, et
d) comparer ladite mesure à une mesure de référence.

Selon un autre objet, l'invention se rapporte à un procédé de *criblage in vitro* ou *ex vivo* d'agents actifs ou de traitements physiques pour le soin de la peau âgée et/ou la prévention et/ou le traitement des signes cutanés du vieillissement, associé(e)s ou non à une sécheresse cutanée, susceptibles de moduler l'activité, l'expression ou la maturation d'une séquence d'acides aminés représentée par une séquence choisie parmi SEQ ID NO: 11, SEQ ID NO : 12, SEQ ID NO : 13 et SEQ ID NO : 16, ou d'une séquence d'acides nucléiques représentée par une séquence choisie parmi SEQ ID NO: 1, SEQ ID NO : 2, SEQ ID NO : 3 et SEQ ID NO : 6, comprenant au moins les étapes consistant à :

a) disposer ladite séquence d'acides aminés ou ladite séquence d'acides nucléiques dans des conditions propices à l'activité, l'expression ou la maturation desdites séquences,
b) mettre en contact ladite séquence d'acides aminés ou ladite séquence d'acides nucléiques avec au moins un agent actif à tester, ou exposer ladite séquence d'acides aminés ou ladite séquence d'acides nucléiques avec un traitement physique à tester,
c) effectuer une mesure qualitative ou quantitative de l'expression, de la maturation ou de l'activité de ladite séquence d'acides aminés ou de ladite séquence d'acides nucléiques, et
d) comparer ladite mesure à une mesure de référence, et
e) observer une différence entre ladite mesure et la mesure de référence pour caractériser l'agent actif ou le traitement comme étant utile pour prévenir et/ou traiter la peau âgée et/ou les signes cutanés du vieillissement, associé(e)(s) ou non à une sécheresse cutanée.

Selon encore un autre mode de réalisation, la présente invention concerne un peptide isolé représenté par la séquence d'acides aminés SEQ ID NO: 18.

La présente invention a pour avantage de proposer un nouveau biomarqueur sensible et spécifique, d'un état de la peau âgée et/ou des signes du vieillissement cutané, associé(e)(s) ou non à une sécheresse cutanée.

Egalement, le présent brevet a pour avantage de proposer un nouveau biomarqueur sensible et spécifique de l'hydratation de la peau, et en particulier de la peau sèche ou des signes de sècheresse cutanée.

L'observation de la présence de la Dermcidine dans le *stratum corneum,* et plus particulièrement de peptides issus spécifiquement de cette protéine, rend avantageusement possible une détermination quantitative ou qualitative de l'expression ou de l'activité de cette protéine, ou des peptides correspondant, par simple prélèvement topique.

La méthode de prélèvement peut par exemple être une technique de type stripping consistant à appliquer sur l'épiderme considéré une portion de ruban adhésif. En décollant ce ruban adhésif, une fraction de la surface de la peau est prélevée. Après extraction protéique, celle-ci peut être ensuite analysée par des méthodes conventionnelles, telles que le dosage immuno-enzymatique ELISA ou une analyse Western-Blot, ou plus particulièrement par la méthode de protéomique différentielle iTRAQ telle que définie ci-après.

Egalement, la présente invention a pour avantage de pouvoir mettre à disposition un nouveau biomarqueur convenant au criblage de nouveaux agents actifs ou de nouveaux traitements physiques convenant à la prévention et/ou au traitement de la peau âgée et/ou des signes cutanés du vieillissement, associé(e)(s) ou non à une sécheresse cutanée. Egalement, le présent brevet a pour avantage de pouvoir mettre à disposition un nouveau biomarqueur convenant au criblage de nouveaux agents actifs ou de nouveaux traitements physiques utiles pour favoriser et/ou renforcer l'hydratation de la peau. De manière préférée, de tels agents ou traitement peuvent convenir à la prévention et/ou au traitement de la peau sèche et/ou des signes de sècheresse cutanée.

Egalement, selon un autre avantage, la présente invention permet de proposer de nouveaux agents actifs convenant à la prévention et/ou au traitement des peaux âgées et/ou des signes cutanés du vieillissement, associé(e)(s) ou non à une sécheresse cutanée.

<u>Séquences d'acides aminés et d'acides nucléiques</u>

La Dermcidine ou Preproteolysin (DCD) est une protéine de 110 acides aminés (SEQ ID NO: 11) comportant un peptide signal de 19 acides aminés (SEQ ID NO: 14) dont le gène est localisé sur le chromosome 12, locus 12q13.1. Deux isoformes de cette protéine sont recensées, l'une plus courte, isoforme 1 (SEQ ID NO: 12), l'autre plus longue, isoforme 2 (SEQ ID NO: 13). Elle est secrétée dans la sueur (Schittek, *Hipfel et al.* 2001).

Après clivage du peptide signal, la Dermcidine (DCD) est une protéine de 90 acides aminés selon la séquence SEQ ID NO : 16.

Après une maturation post-traductionnelle protéolytique, essentiellement par la Cathepsine D, qui a lieu dans la sueur (Baechle, Flad *et al.,* 2006), ce précurseur protéique donne naissance à la DCD-1 (SEQ ID NO: 19) et à la DCD-1L (SEQ ID NO :20) ainsi qu'à de nombreux peptides dont la longueur varie de 25 à 48 aa (Flad, Bogumil *et al.,* 2002).

Sauf indication contraire, le terme « Dermcidine » vise à désigner dans le présent brevet les séquences d'acides aminés représentées par SEQ ID NO: 11, SEQ ID NO: 12, SEQ ID NO: 13, SEQ ID NO: 16, SEQ ID NO: 19 et SEQ ID NO: 20, ayant ou non subi une maturation post-traductionnelle.

Selon un mode de réalisation préféré, on entend désigner par Dermcidine plus particulièrement la séquence d'acides aminés représentée par SEQ ID NO: 16.

Parmi les peptides issus de la maturation de la Dermcidine, comme décrit dans US 2008/020976, certains montrent des activités antimicrobiennes contre différents microorganismes, tel que *Staphylococcus aureus, Escherichia coli, Enterococcus faecalis*, ou *Candida albicans.* Les peptides antimicrobiens sont dérivés de la partie C-terminale de la protéine.

La partie N-terminale de la protéine (SEQ ID NO: 15) est constituée d'un facteur biologiquement actif favorisant la survie cellulaire (Cunningham, Hodge *et al.* 1998).

Cette protéine est exprimée de façon constitutive dans la glande sudoripare (Rieg, Garb *et al.* 2004), et est impliquée dans les voies de signalisation de type PI3K/AKT/mTOR particulièrement importante dans les phénomènes de prolifération et de survie cellulaire (Moreira, Strauss *et al.* 2008). La diminution de la DCD observée dans la sueur des individus souffrant d'atopie pourrait expliquer en partie leur susceptibilité microbienne (Rieg, Steffen *et al.* 2005).

Certains des fragments antimicrobiens ainsi que de la partie N-terminale sont capables de stimuler la production de cytokines/chemokines par des kératinocytes ou d'autres types cellulaires l'impliquant dans la régulation de l'immunité cutanée (Watchorn, Dowidar *et al.* 2005 ; Niyonsaba, Suzuki *et al.* 2009). Il a récemment été proposé l'utilisation de la DCD comme un marqueur spécifique particulièrement sensible de la présence de traces de sueur par RT-PCR ou par ELISA utilisable dans le domaine médico-légal (Sakurada, Akutsu *et al.* 2009).

Une séquence d'acides aminés telle que décrite dans le présent brevet peut être codée par une séquence d'acides nucléiques représentées par SEQ ID NO: 1, ou être un analogue ou un fragment de cette séquence d'acides aminés.

Par « analogue d'une séquence d'acides aminés », on entend désigner toute séquence d'acides aminés ayant une identité de séquence d'au moins 85 %, de préference d'au moins 90 %, et plus préférentiellement d'au moins 95 % avec ladite séquence, et une activité biologique de même nature.

Par « activité biologique de même nature » à l'égard d'une séquence d'acides aminés, on peut entendre les propriétés antimicrobiennes ou de stimulation de la survie et/ou de la prolifération cellulaire habituellement attribuées à la Dermcidine. De préference, par « activité biologique de même nature » à l'égard d'une séquence d'acides aminés, on entend les propriétés de prévention et/ou de traitement à l'égard de la peau âgée ou des signes du vieillissement cutané, associé(e)(s) ou non à une sécheresse cutanée, voire à l'égard de la peau sèche et/ou des signes de sècheresse cutanée.

L'identité de séquence peut être déterminée par comparaison visuelle ou au moyen de tout outil informatique généralement utilisé dans le domaine, tel que les programmes BLAST disponibles sur www.ncbi.nlm.nih.gov et utilisés avec les paramètres configurés par défaut.

Un analogue peut être un agent peptidomimétique.

Un analogue d'une séquence d'acides aminés peut résulter de modifications issues de mutation ou de variation dans les séquences des peptides provenant soit de la délétion ou de l'insertion d'un ou plusieurs acides aminés, soit de la substitution d'un ou plusieurs acides aminés, soit encore d'un épissage alternatif. Plusieurs de ces modifications peuvent être combinées.

Avantageusement, un analogue d'une séquence d'acides peut comprendre des substitutions conservatrices par rapport à cette séquence d'acides aminés.

A titre d'exemple de mutations que l'on peut considérer dans le présent brevet, il peut être mentionné, de manière non exhaustive, le remplacement d'un ou plusieurs résidus d'acides aminés par des résidus d'acides aminés ayant un indice hydropathique similaire sans pour autant affecter de manière sensible les propriétés biologiques du polypeptide. L'indice hydropathique est un indice attribué aux acides aminés en fonction de leur hydrophobicité et de leur charge (Kyte et al. (1982), J. Mol. Biol., 157 : 105).

Une séquence d'acides aminés ou un analogue de celle-ci peut être une séquence d'acides aminés ayant subi une ou plusieurs maturation(s) post-traductionnelle(s).

Par « maturation(s) post-traductionnelle(s) », on entend englober l'ensemble des modifications qu'une séquence d'acides aminés est susceptible de subir à l'issue de sa synthèse dans une cellule, telle que par exemple une ou des phosphorylation(s), une ou des thiolation(s), une ou des acétylation(s), une ou des glycosylation(s), une ou des lipidation(s), telles qu'une farnésylation ou une palmitoylation, un réarrangement structural de type formation de ponts disulfures et/ou de type clivage à l'intérieur de la séquence peptidique.

Un analogue d'une séquence d'acides aminés présente, par ailleurs, sensiblement la même activité biologique que cette séquence d'acides aminés.

Il est par ailleurs connu qu'une séquence primaire d'acides aminés, peut comprendre des sites spécifiquement reconnus par des enzymes de type protéase, telles que la trypsine, qui, une fois la reconnaissance de ces sites effective vont induire le clivage de la séquence par protéolyse. Cette protéolyse résulte en la génération de divers peptides, ou fragments de séquences d'acides aminés.

En conséquence, le brevet décrit également des fragments de la Dermcidine, issus le cas échéant de sa protéolyse.

Au sens du brevet, on entend par « fragment d'une séquence d'acides aminés » toute portion de la séquence d'acides aminés comprenant de 3 à 48 acides aminés consécutifs de ladite séquence, de préférence de 6 à 36, de préférence 8 à 32, et plus préférentiellement de 10 à 30 acides aminés consécutifs de ladite séquence, et a une activité biologique de même nature.

Une séquence d'acides aminés peut être une séquence d'acides aminés représentée par une séquence choisie parmi SEQ ID NO: 11 à 20, notamment parmi SEQ ID NO: 11, SEQ ID NO: 12, SEQ ID NO: 13, un analogue, ou un fragment de celle-ci.

Une séquence d'acides aminés peut être une séquence d'acides aminés représentée par une séquence choisie parmi SEQ ID NO: 14, SEQ ID NO: 15, SEQ ID NO: 16,
SEQ ID NO: 17, SEQ ID NO: 18, SEQ ID NO : 19, SEQ ID NO : 20, un analogue, ou un fragment de celle-ci.

Une séquence d'acides aminés peut être une séquence d'acides aminés représentée par une séquence choisie parmi SEQ ID NO: 16, SEQ ID NO: 17, ou SEQ ID NO: 18, SEQ ID NO: 19 ou SEQ ID NO : 20, un analogue ou un fragment de celle-ci.

Une séquence d'acides aminés peut être une séquence d'acides aminés représentés par une séquence choisie parmi SEQ ID NO : 17, SEQ ID NO : 18, SEQ ID NO : 19 ou SEQ ID NO : 20, un analogue ou un fragment de celle-ci.

Une séquence d'acides aminés peut être une séquence d'acides aminés représentée par une séquence choisie parmi SEQ ID NO: 17, SEQ ID NO: 18, un analogue ou un fragment de celle-ci.

Un polypeptide peut également être une séquence d'acides aminés naturelle ou synthétique, le cas échéant susceptible d'être obtenue après lyse enzymatique ou chimique de la Dermcidine ou par synthèse chimique ou biologique ou par extraction à partir d'un tissu biologique, comme par exemple la peau, exprimant naturellement cette séquence d'acides aminés ou après transfection de celui-ci, ainsi que les différentes formes post-traductionnelles de celui-ci, ou encore toute séquence d'acides aminés naturelle ou synthétique dont la séquence comprend totalement ou partiellement une séquence d'acides aminés précitée, par exemple les variants et les analogues.

L'homme de l'art peut obtenir une séquence d'acides aminés conforme à l'invention au moyen de procédés à base d'ADN recombinant, comme par exemple, ceux décrits dans le manuel « Molecular Cloning - A Laboratory Manual » (2ème édition), Sambrook et al., 1989, Vol. I-III, Coldspring Harbor Laboratory, Coldspring Harbor Press, NY, (Sambrook).

Selon un objet, le présent brevet décrit en tant que tel un peptide isolé représenté par une séquence d'acides aminés choisie parmi SEQ ID NO: 17 ou SEQ ID NO: 18, un analogue ou un fragment de celle-ci.

L'invention se rapporte en particulier à un peptide isolé représenté par la séquence d'acides aminés SEQ ID NO: 18.

Selon un autre mode de réalisation, une séquence d'acides aminés peut également être une séquence d'acides aminés telle que définie précédemment, fusionnée avec une autre séquence d'acides aminés, un agent de ciblage hydrophile ou hydrophobe, un précurseur de bio-conversion, un agent de marquage luminescent, radioactif ou colorimétrique.

De manière non limitative, on peut citer comme exemple de composés susceptibles d'être couplés à une séquence d'acides aminés conforme à l'invention des protéines fluorescentes comme la « Green Fluorescent Protein », des composés chimiques fluorescents, tels que la rhodamine, la fluorescéine, ou le Texas Red®, des composés phosphorescents, des éléments radioactifs, tels que $^{3}H$, $^{14}C$, $^{35}S$, $^{121}I$ ou $^{125}I$, ou des agents de marquage colorimétrique comme les substrats chromogènes sensibles à l'action de la galactosidase, de la peroxydase, de la chloramphénicol acétyltransférase, de la luciférase ou de la phosphatase alcaline.

Selon la nature des composés susceptibles d'être couplés avec une séquence d'acides aminés selon le brevet, le couplage peut être opéré par des procédés chimiques, notamment au moyen de fonctions chimiques réactives ou par des procédés de biologie moléculaire connus de l'homme de l'art.

Avantageusement, une séquence d'acides aminés selon le brevet peut être codée par une séquence d'acides nucléiques choisie parmi une séquence représentée par SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, un analogue ou un fragment de celle-ci.

Le présent brevet décrit également des séquences d'acides nucléiques codant pour une séquence d'acides aminés et leur mise en oeuvre dans les différentes utilisations et procédé du brevet.

Ainsi, le présent brevet décrit également à une séquence d'acides nucléiques, notamment d'acides désoxyribonucléiques, ou d'acides ribonucléiques, représentée par SEQ ID NO: 1, un analogue ou un fragment de celle-ci.

Au sens du présent brevet, on entend par « fragment de séquence d'acides nucléiques », une séquence d'acides nucléiques comprenant de 9 à 144 paires de bases consécutives de ladite séquence, de préférence de 18 à 108, de préférence 24 à 96, et plus préférentiellement de 30 à 90 paires de bases consécutives de ladite séquence, et codant pour une séquence d'acides aminés ayant une activité biologique de même nature que la séquence d'acides aminés codée par ladite séquence.

Au sens du présent brevet, on entend par « analogue d'une séquence d'acides nucléiques » une séquence d'acides nucléiques ayant une identité de séquence d'au moins 85 %, de préférence d'au moins 90 %, et plus préférentiellement d'au moins 95 % avec ladite séquence, et codant pour une séquence d'acides aminés ayant une activité biologique de même nature que la séquence d'acides aminés codée par ladite séquence.

Par « analogue d'une séquence d'acides nucléiques », on entend désigner une séquence d'acides nucléiques, éventuellement résultant de la dégénérescence du code des acides nucléiques, et codant pour une séquence d'acides aminés selon le brevet, notamment tel que défini précédemment.

Une séquence d'acides nucléiques selon le brevet peut être représentée par une séquence choisie parmi SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO : 9 et SEQ ID NO : 10, un analogue ou un fragment de celle-ci.

Une séquence d'acides nucléiques selon le brevet peut être de toutes origines possibles, à savoir soit animale, en particulier de mammifères et encore plus particulièrement humaine, soit végétale, soit de micro-organismes, tels que par exemple virus, phages, ou bactéries entre autres, ou encore de champignons, sans préjuger du fait qu'elles soient présentes de manière naturelle ou non dans ledit organisme d'origine.

Le brevet décrit également des séquences d'acides nucléiques isolées et purifiées codant pour une séquence d'acides aminés considérés selon le brevet, ainsi qu'à leurs analogues et fragments.

Une séquence d'acides nucléiques selon le brevet peut comprendre une séquence sens, anti-sens ou interférentielle correspondant à une séquence codant pour un polypeptide selon le brevet.

Le brevet a également pour objet des séquences d'acides nucléiques, notamment d'acides ribonucléiques ou désoxyribonucléiques, comprenant une séquence sens ou anti-sens, notamment « small interferent RNA » (siRNA), correspondant au moins à une séquence codant pour un polypeptide de l'invention ou une séquence d'acides nucléiques représentée par SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO : 9, SEQ ID NO : 10, un analogue ou un fragment de celle-ci.

Peau âgée et signes cutanés du vieillissement

Par « peau âgée », on entend un état général de la peau résultant du vieillissement chronologique et/ou du vieillissement photoinduit.

Par « signes cutanés du vieillissement », on entend toutes les modifications de l'aspect extérieur de la peau dues au vieillissement qu'il soit d'origine chronologique et/ou photo-induit.

A titre d'exemple de ces modifications considérées dans l'invention, on peut citer les rides et ridules, la peau flétrie, le manque d'élasticité et/ou de tonus de la peau, l'amincissement du derme et/ou la dégradation des fibres de collagène, ce qui entraîne l'apparence de peau molle et ridée.

On entend également toutes les modifications internes de la peau qui ne se traduisent pas systématiquement par un aspect extérieur modifié, comme par exemple toutes les dégradations internes de la peau, et plus particulièrement la dégradation des fibres d'élastine, ou fibres élastiques, consécutives à une exposition aux rayonnements ultra-violets.

En particulier, les signes cutanés du vieillissement visés par l'invention sont choisis parmi un amincissement de la peau, une perte de fermeté, une perte d'élasticité, une perte de densité, ou une perte de tonicité de la peau, une sécheresse de la peau, l'apparition d'un microrelief de la peau marqué, la formation et/ou la présence de ridules et/ou de rides, une altération de l'éclat du teint de la peau, un aspect papyracé de la peau, une altération de l'odeur de la peau, un affaissement de la peau, et un flétrissement de la peau.

De manière préférée, les signes cutanés du vieillissement visés par l'invention sont choisis parmi un amincissement de la peau, l'apparition d'un microrelief de la peau marqué, la formation et/ou la présence de ridules et/ou de rides, un affaissement de la peau, et un flétrissement de la peau.

De manière préférée encore, les signes cutanés du vieillissement visés par l'invention sont choisis parmi l'apparition d'un microrelief de la peau marqué, la formation et/ou la présence de ridules et/ou de rides, un affaissement de la peau, et un flétrissement de la peau.

On entend par « hydratation de la peau », l'ensemble des mécanismes cellulaires et moléculaires qui conduisent à assurer et à maintenir la présence d'une quantité d'eau physiologique dans l'épiderme et le derme, ainsi que la quantité d'eau résultante.

Selon un aspect, le brevet vise à maintenir voire à stimuler l'homéostasie de ces mécanismes, et ainsi à favoriser et/ou renforcer l'hydratation de la peau. Ainsi, selon un aspect, le brevet s'adresse aux peaux présentant un état hydraté physiologique, également qualifié de normal.

Selon un autre aspect, le brevet vise à restaurer l'équilibre ou à réduire le risque de survenue d'un déséquilibre dans l'homéostasie de ces mécanismes, et ainsi prévenir et/ou traiter la peau sèche et/ou les signes de sècheresse cutanée.

Par « signes de sécheresse cutanée », on entend toutes les modifications de l'aspect de la peau dues à un déficit en eau dans l'épiderme et/ou le derme.

Selon le degré de manifestation du déficit en eau de la peau, une peau sèche peut apparaître rugueuse au toucher, ridée, voire couverte de squames. Une peau sèche peut se manifester, pour l'essentiel, par une sensation de tiraillements et/ou de tension.

Par « peau séche », on entend un état général de la peau résultant d'un déficit en eau de l'épiderme et/ou du derme.

Une peau sèche peut se manifester par un trouble de la desquamation et présenter différents stades en fonction de la sévérité de cette desquamation.

Lorsque la peau est légèrement sèche, ces squames sont abondantes mais peu visibles à l'oeil nu, l'élimination s'effectue cornéocyte par cornéocyte. Elles sont de moins en moins nombreuses mais de plus en plus visibles à l'oeil nu lorsque ce désordre s'aggrave, les amas peuvent comprendre plusieurs centaines de cornéocytes, représentant ainsi des amas plus ou moins grands, appelées squames.

La sécheresse cutanée peut être constitutionnelle ou acquise.

Dans le cas de la peau sèche constitutionnelle, on peut distinguer deux catégories : les peaux pathologiques et les peaux non pathologiques.

Les peaux sèches constitutionnelles pathologiques sont essentiellement représentées par la dermatite atopique et les ichthyoses. Elles sont quasiment indépendantes des conditions extérieures et ont pour origine des modifications génétiques connues ou inconnues. Parmi les modifications génétiques connues affectant l'hydratation cutanée on peut citer par exemple des modifications du gène de la Transglutaminase-1 ou celles du gène de la Filaggrine.

Est également décrite une composition pharmaceutique ou dermatologique comprenant dans un milieu physiologiquement acceptable une quantité efficace (i) d'au moins une séquence d'acides aminés selon le brevet, ou (ii) d'au moins une séquence d'acides nucléiques selon le brevet, ou (iii) d'au moins un agent modulateur de l'activité, de l'expression ou de la maturation de ladite séquence d'acides aminés ou de ladite séquence d'acides nucléiques, à titre d'agent actif pour prévenir et/ou traiter les peaux sèches constitutionnelles pathologiques choisies parmi la dermatite atopique et les ichtyoses.

Dans le cas des peaux sèches constitutionnelles non pathologiques, la sévérité de l'état de sécheresse peut, pour sa part, dépendre de facteurs extérieurs. Rentrent dans cette catégorie de peau, la peau sénile (caractérisée par une diminution générale du métabolisme cutané avec l'âge), la peau fragile (très sensible aux facteurs extérieurs et souvent accompagnée d'érythème et de rosacée) et la xérose vulgaire (d'origine génétique probable et se manifestant en priorité sur le visage, les membres et le dos des mains).

Dans le cas de peau sèche acquise, l'intervention de paramètres extérieurs tels que l'exposition aux agents chimiques, à des conditions climatiques difficiles, aux rayons solaires ou bien encore certains traitements thérapeutiques (rétinoïdes, par exemple) est déterminante. Sous ces influences extérieures, l'épiderme peut devenir alors momentanément et localement sec. Cela peut concerner tout type d'épiderme.

Quelle qu'en soit l'origine, une peau atteinte de sécheresse cutanée peut présenter, généralement, les signes suivants : aspect rugueux au toucher et écailleux, ainsi qu'une souplesse et une élasticité diminuées.

La peau sèche, également dénommée « xérose », peut apparaître à n'importe quel âge, et n'être pas liée à une condition pathologique. On parlera dans ce cas de sécheresse « acquise ».

Cependant, la xérose devient plus fréquente et gênante avec l'âge, notamment chez les femmes. On parle alors de xérose sénile. Par ailleurs, les femmes subissent généralement une aggravation de l'assèchement cutané lors de la ménopause, probablement du fait du dérèglement hormonal caractéristique de ce phénomène. Les zones les plus affectées sont la partie inférieure des jambes, la partie dorsale des avant-bras et les mains.

Comme mentionné précédemment, la sécheresse acquise peut subir l'influence de facteurs extérieurs. Par exemple, l'apparition de la peau sèche peut être favorisée par un temps froid, sec et hivernal. On parle alors de xérose hivernale. La sécheresse cutanée peut également être induite par un stress exogène, d'origine chimique, par exemple de type détergent anionique, ou encore d'origine mécanique (frottement, rasage).

Une utilisation cosmétique selon le brevet peut avantageusement convenir pour prévenir et/ou traiter les peaux sèches séniles ou fragiles, ou la xérose, notamment choisie parmi la xérose vulgaire, la xérose sénile, et la xérose hivernale.

Bien qu'aucune étude n'ait démontré une incidence de la sécheresse sur l'origine et la formation des rides et ridules qui sont essentiellement attribuables au vieillissement, sur le plan visuel, une peau sèche rend celles-ci plus apparentes. Une peau sèche peut donc être associée à une peau âgée présentant des rides ou ridules.

Par ailleurs, sur le plan sensoriel, la sécheresse cutanée est caractérisée par une sensation de tiraillements et/ou de démangeaisons. Pour des raisons évidentes, ces manifestations sont non seulement source d'inconforts, voire de douleurs, mais également d'une apparence inesthétique.

Est également décrite une utilisation cosmétique pouvant avantageusement convenir pour prévenir et/ou traiter les sensations de tiraillements et/ou de démangeaisons associées aux peaux sèches.

A titre d'exemple de signes de sècheresse cutanée considérés dans le brevet, on peut citer la peau flétrie, le manque d'élasticité, de souplesse et/ou de tonus de la peau, le toucher rugueux, la présence de crevasse, la desquamation, la présence d'écailles, ou les rides et ridules associées à la peau sèche.

Les signes de sècheresse cutanée considérés sont également toutes les modifications internes de la peau qui ne se traduisent pas systématiquement par un aspect extérieur modifié, comme par exemple toutes les dégradations internes de la peau, et plus particulièrement la dégradation des fibres d'élastine, ou fibres élastiques, consécutives à un assèchement du derme. Selon un aspect , les signes de sècheresse cutanée sont choisis parmi une peau flétrie, un manque d'élasticité, de souplesse et/ou de tonus de la peau, un toucher rugueux, une présence de crevasse, une desquamation, une présence d'écailles, des rides et ridules associées à la peau sèche, et une sensation de tiraillements et/ou de démangeaisons.

Selon l'invention, la peau âgée ou les signes cutanés du vieillissement peuvent être associé(e)s ou non à une sécheresse cutanée.

Biomarqueur

La présente invention concerne l'utilisation d'au moins une séquence d'acides aminés représentée par une séquence choisie parmi SEQ ID NO: 11 à SEQ ID NO: 20, et de préférence est choisie parmi SEQ ID NO: 16, SEQ ID NO: 17, SEQ ID NO: 18, SEQ ID NO: 19, SEQ ID NO: 20, ou d'au moins une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, à titre de biomarqueur d'un état de la peau âgée et/ou des signes cutanés du vieillissement, associé(e)(s) ou non à une sécheresse cutanée.

La présente invention concerne l'utilisation d'au moins une séquence d'acides aminés représentée par une séquence choisie parmi SEQ ID NO: 11 à SEQ ID NO: 20, et de préférence est choisie parmi SEQ ID NO: 16, SEQ ID NO: 17, SEQ ID NO: 18, SEQ ID NO : 19, SEQ ID NO : 20, à titre de biomarqueur d'un état de la peau âgée et/ou des signes cutanés du vieillissement, associé(e)(s) ou non à une sécheresse cutanée.

Dans le présent brevet, est également décrit l'utilisation d'au moins une séquence d'acides aminés selon le brevet, ou d'au moins une séquence d'acides nucléiques selon le brevet, à titre de biomarqueur d'un état d'hydratation de la peau, et plus particulièrement de la peau sèche et/ou des signes de sècheresse cutanée.

De manière préférée, une utilisation conforme au brevet permet de caractériser un état de la peau tel que la peau âgée et/ou les signes cutanés du vieillissement, associé(e)(s) ou non à une sécheresse cutanée.

Selon un mode de réalisation, une diminution de l'activité, de l'expression ou de la maturation dudit biomarqueur peut être indicative d'une peau âgée et/ou de signes cutanés du vieillissement, associé(e)(s) ou non à une sécheresse cutanée.

Selon un mode de réalisation, il est également décrit qu'une diminution de l'activité, de l'expression ou de la maturation dudit biomarqueur peut être indicative d'une peau en déficit d'hydratation, et plus particulièrement d'une peau sèche et/ou de signes de sècheresse cutanée.

Le brevet concerne également l'utilisation d'au moins une séquence d'acides aminés selon le brevet, ou d'au moins une séquence d'acides nucléiques selon le brevet, pour caractériser l'efficacité d'un traitement cosmétique de la peau, et de préférence de la peau âgée et/ou des signes cutanés du vieillissement, associé(e)(s) ou non à une sécheresse cutanée.

Est également décrite l'utilisation d'au moins une séquence d'acides aminés selon le brevet, ou d'au moins une séquence d'acides nucléiques selon le brevet, pour caractériser l'efficacité d'un traitement cosmétique de la peau sèche et/ou des signes de sècheresse cutanée.

Selon un mode de réalisation, une augmentation de l'activité, de l'expression ou de la maturation dudit biomarqueur peut être indicative d'un traitement cosmétique efficace pour exercer un effet bénéfique sur la peau et plus préférentiellement un effet à l'égard de la peau âgée et/ou de signes cutanés du vieillissement, associé(e)(s) ou non à une sécheresse cutanée.

Il est également décrit qu'une augmentation de l'activité, de l'expression ou de la maturation dudit biomarqueur peut être indicative d'un traitement cosmétique efficace pour exercer un effet bénéfique sur la peau en déficit d'hydratation, et plus préférentiellement un effet à l'égard de la peau sèche et/ou de signes de sècheresse cutanée.

Dans une utilisation conforme au brevet, une diminution ou une augmentation de l'activité, de l'expression ou de la maturation dudit biomarqueur peut être déterminée par comparaison à une mesure de référence obtenue selon toute méthode connue de l'homme de l'art.

Une « mesure de référence » au regard d'un paramètre donné, est une mesure qualitative ou quantitative de ce paramètre effectuée dans des conditions dites « contrôles » ou « normales », par exemple déterminée dans un échantillon de référence, ou déterminée dans un échantillon en l'absence d'un traitement présumé avoir un effet sur le paramètre.

Par exemple, une mesure de référence pour une séquence d'acides aminés ou une séquence d'acides nucléiques selon le brevet peut être une valeur quantitative ou qualitative relative à l'expression, la maturation ou l'activité desdites séquences déterminée dans un échantillon de peau jeune et physiologiquement saine, ou déterminée dans un échantillon de peau, notamment de peau âgée, avant un traitement cosmétique.

Egalement par exemple, une mesure de référence pour une séquence d'acides aminés ou une séquence d'acides nucléiques selon le brevet peut être une valeur quantitative ou qualitative relative à l'expression, la maturation ou l'activité desdites séquences déterminée dans un échantillon de peau physiologiquement saine et normalement hydratée, ou déterminée dans un échantillon de peau sèche, avant un traitement cosmétique.

De préférence, une mesure de référence est une mesure statistique, c'est à dire ayant été répétée sur différents échantillons de sorte à obtenir une moyenne.

La mesure de référence peut être effectuée parallèlement ou séquentiellement à la mesure test.

Elle peut également être une mesure « historique », c'est-à-dire effectuée antérieurement à la mesure test, et stockée, par exemple dans une base de données, en vue d'une utilisation ultérieure.

Une comparaison de la mesure test à une mesure de référence, et une observation d'une déviation ou d'une absence de déviation entre les deux mesures, permet de tirer une information quant au paramètre mesuré, par exemple la diminution ou l'augmentation l'expression, de la maturation ou de l'activité d'une séquence d'acides aminés ou d'une séquence d'acides nucléiques selon le brevet.

Une telle information peut être ultérieurement mise en oeuvre pour déterminer le caractère jeune ou âgée d'une peau. Egalement, une telle information peut être ultérieurement mise en oeuvre pour déterminer le caractère normalement hydraté ou sec d'une peau

Un procédé selon le brevet peut également être effectué sur un échantillon de peau, prélevé à partir d'un modèle cellulaire épidermique, ou d'une peau isolée reconstruite afin d'en qualifier l'état.

Selon un mode de réalisation, le brevet concerne un procédé, en particulier *in vitro* ou *ex vivo,* pour caractériser un état de la peau.

Il est également décrit un procédé, en particulier *in vitro* ou *ex vivo,* pour caractériser un état d'hydratation de la peau.

Un procédé selon le brevet permet avantageusement de caractériser un état âgé de la peau et/ou des signes du vieillissement cutané, et plus particulièrement de caractériser un état âgé de la peau d'origine chronologique et/ou photo-induit.

Il est également décrit qu'un procédé selon le brevet permet avantageusement de caractériser un état sec de la peau et/ou des signes de sécheresse cutanée.

L'invention concerne un procédé cosmétique *in vitro* ou *ex vivo,* pour caractériser l'efficacité d'un traitement cosmétique de la peau âgée et/ou des signes cutanés du vieillissement, associé(e)(s) ou non à une sécheresse cutanée, chez un individu en ayant besoin, comprenant au moins les étapes consistant à :

a) effectuer, avant la mise en oeuvre du traitement cosmétique, dans un premier échantillon de peau isolé prélevé chez ledit individu, au moins une première mesure qualitative ou quantitative de l'expression, de la maturation ou de l'activité d'au moins une séquence d'acides aminés choisie parmi SEQ ID NO: 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20 ou d'au moins une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10,

b) effectuer, après la mise en oeuvre du traitement cosmétique, dans un second échantillon de peau isolé prélevé chez ledit individu, au moins une seconde mesure, qualitative ou quantitative de l'expression, de la maturation ou de l'activité de ladite séquence d'acides aminés ou de ladite séquence d'acides nucléiques, et

c) comparer les première et deuxième mesures, notamment afin d'en déduire une information relative à un effet au moins de la mise en oeuvre du traitement cosmétique.

Selon un mode de réalisation particulier, la séquence d'acides aminées est représentée parmi une séquence choisie parmi SEQ ID NO : 17 et SEQ ID NO : 18.

Il va de soi que les mesures effectuées aux étapes a) et b) doivent être comparables l'une à l'autre, et donc être relatives au même paramètre.

De manière préférée, ce procédé de l'invention permet de mettre en évidence un effet d'un traitement cosmétique susceptible de faire régresser la peau âgée et/ou les signes cutanés du vieillissement, associé(e)(s) ou non à une sécheresse cutanée.

Il est également décrit un procédé cosmétique, ou non thérapeutique, en particulier *in vitro* ou *ex vivo,* pour caractériser l'efficacité d'un traitement cosmétique de la peau sèche et/ou des signes de sècheresse cutanée, chez un individu en ayant besoin, comprenant au moins les étapes consistant à :

a) effectuer, avant la mise en oeuvre du traitement cosmétique, dans un premier échantillon de peau isolé prélevé chez ledit individu, au moins une première mesure qualitative ou quantitative de l'expression, de la maturation ou de l'activité d'une séquence d'acides aminés selon le brevet ou d'une séquence d'acides nucléiques selon le brevet,
b) effectuer, après la mise en oeuvre du traitement cosmétique, dans un second échantillon de peau isolé prélevé chez ledit individu, au moins une seconde mesure, qualitative ou quantitative de l'expression, de la maturation ou de l'activité de ladite séquence d'acides aminés selon le brevet ou de ladite séquence d'acides nucléiques selon le brevet, et
c) comparer les première et deuxième mesures, notamment afin d'en déduire une information relative à un effet au moins de la mise en oeuvre du traitement cosmétique.

Il va de soi que les mesures effectuées aux étapes a) et b) doivent être comparables l'une à l'autre, et donc être relatives au même paramètre.

De manière préférée, il est également décrit qu'un procédé de l'invention permet de mettre en évidence un effet d'un traitement cosmétique susceptible de faire régresser la peau sèche et/ou les signes de sècheresse cutanée.

La mesure qualitative ou quantitative de l'expression, de la maturation ou de l'activité d'une séquence d'acides nucléiques décrite par le brevet peut être déterminée par toute méthode connue de l'homme de l'art.

A titre d'exemple de méthodes convenant à l'invention, il peut être fait mention de la réaction de polymérisation en chaîne (PCR), quantitative (Q-PCR) ou non, en présence ou non de transcriptase inverse (RT-PCR ou Q-RT-PCR), du Northern-blot, de la méthode « ribonuclease protection assay », des méthodes avec des puces à ADN, des méthodes avec des puces transcriptomiques, des méthodes avec des puces à oligonucléotides, des méthodes d'hybridation *in situ.*

A titre d'exemple d'agents convenant à la détection d'une séquence d'acides nucléiques selon le brevet, et en particulier d'une séquence d'ARNm, il peut être fait mention de sondes d'acides nucléiques marquées pouvant s'hybrider à une séquence d'acides nucléiques selon le brevet.

Une telle sonde d'acide nucléique peut être aisément obtenue par toute méthode connue de l'homme de l'art.

Ainsi, les séquences d'acides nucléiques décrites par le brevet peuvent être utilisées pour réaliser des amorces oligonucléotidiques sens et/ou anti-sens, qui s'hybrident dans des conditions de forte stringence à au moins une des séquences SEQ ID NO: 1, SEQ ID NO: 2, SEQ ID NO: 3, SEQ ID NO: 4, SEQ ID NO: 5, SEQ ID NO:6, SEQ ID NO: 7, SEQ ID NO: 8, SEQ ID NO : 9, SEQ ID NO : 10, un analogue ou un fragment celle-ci.

L'expression d'une séquence d'acide nucléique peut également être déterminée, de manière indirecte, par la détermination de l'expression de la séquence d'acides aminées codée par ladite séquence, au moyen de toute technique connue dans le domaine, telle que le Western-Blot, l'ELISA, la méthode de BRADFORD ou de LOWRY, ou comme indiqué ci-après.

La mesure qualitative ou quantitative, de l'expression, de la maturation, ou de l'activité d'une séquence d'acides aminés peut être effectuée au moyen de toute méthode connue de l'homme de l'art.

A titre de méthodes de détection de l'expression, de la maturation, ou de l'activité d'une séquence d'acides aminés, il peut être fait mention du Western-blot, du Slot-blot, du Dot-blot, des méthodes ELISA (Enzyme Linked Immuno-Sorbent Assay) de type singleplex ou multiplex, des méthodes de protéomique ou de glycomique, de coloration de polypeptides dans un gel de polyacrylamide par un colorant à base d'Argent, par le bleu de Coomassie ou par le SYPRO, de l'immunofluorescence, de l'absorption UV, de méthodes immunohistochimiques en microscopie classique, électronique ou confocale, de FRET (fluorescence resonance energy transfer/transfert d'énergie par résonance de fluorescence), des méthodes de TR-FRET (time resolved FRET/FRET en résolution de temps), des méthodes de FLIM (fluorescence lifetime imaging microscopy/imagerie par microscopie en temps de fluorescence), des méthodes de FSPIM (fluorescence spectral imaging microscopy/imagerie par microscopie en spectre de fluorescence), des méthodes de FRAP (fluorescence recovery after photobleaching/recouvrement de fluorescence après photoblanchiment), des méthodes par gène rapporteur, des méthodes d'AFM (atomic force microscopy/microscopie par force atomique), des méthodes de résonance plasmonique de surface, des méthodes de microcalorimétrie, des méthodes de cytométrie en flux, des méthodes de biosenseurs, des méthodes de radioimmuno-essais (RIA), des méthodes de focalisation isoélectrique, et des tests enzymatiques, des méthodes mettant en oeuvre des puces à peptides, des puces à sucre, des puces d'anticorps, des méthodes de spectrométrie de masse, des méthodes de spectrométrie de type SELDI-TOF (Ciphergen).

De façon plus générale, des méthodes de dosage immunoenzymatiques à partir de solutions de protéines, plus quantitatives et sensibles peuvent en particulier être utilisées. Ces méthodes de type ELISA associent des couples d'anticorps de capture et de détection spécifique de l'antigène ciblé. Des anticorps commerciaux ou des anticorps polyclonaux, monoclonaux ou recombinants développés spécifiquement peuvent être utilisés. Des techniques ELISA multiplexes de grande capacité peuvent également être mises en oeuvre. On peut ainsi citer l'approche multiplexe de type anticorps sur billes Luminex (par exemple Bioplex de Bio-Rad), de type anticorps sur surface plane (« antibodies-arrays ») (par exemple approche proposée par la société MesoScale Discovery).

En particulier, il peut être avantageux de détecter l'expression d'une séquence d'acides aminés, au moyen d'un anticorps, le cas échéant sous une forme marquée. Un tel anticorps peut être marqué au moyen d'une substance détectable directement ou détectable par réaction avec un autre réactif.

Par « anticorps », on entend désigner de manière générale des anticorps monoclonaux ou polyclonaux, ainsi que des fragments d'immunoglobuline susceptibles de lier un antigène et qui peuvent être produits par toute technique de génie génétique connue de l'homme de l'art ou par coupure enzymatique ou chimique d'anticorps intact.

Un anticorps susceptible d'être utilisé à titre d'outil d'évaluation d'un état d'un épiderme peut être obtenu par tout procédé connu de l'homme de l'art, tel que décrit dans « Antibodies: A Laboratory Manual », Cold Spring Harbor Laboratory Press, Cold Spring Harbor, N.Y. (1990).

Selon un mode de réalisation préféré, il peut être avantageux de détecter l'expression d'une séquence d'acides aminés de l'invention au moyen d'une méthode protéomique différentiel « iTRAQ ».

Selon un mode de réalisation préféré, il peut être également avantageux de détecter l'expression d'une séquence d'acides aminés de l'invention au moyen d'un Western Blot.

Une telle méthode est connue de l'homme de l'art et peut avantageusement être mise en oeuvre comme décrit dans les exemples ci-après.

De manière particulière, par « activité » à l'égard d'une séquence d'acides aminés on entend une activité antimicrobienne, une activité de stimulation de la survie cellulaire, une activité de stimulation de la prolifération cellulaire, ou une activité de réduction ou de prévention de la peau âgée et/ou des signes cutanés du vieillissement, associé(e)(s) ou non à une sécheresse cutanée, comme indiqué ci-dessus.

Egalement de manière particulière, par « activité » à l'égard d'une séquence d'acides aminés on entend une activité antimicrobienne, une activité de stimulation de la survie cellulaire, une activité de stimulation de la prolifération cellulaire, ou une activité de réduction ou de prévention de la peau sèche et/ou des signes de sècheresse cutanée, comme indiqué ci-dessus.

Une telle activité peut être déterminée par toute méthode connue de l'homme de l'art, comme par exemple, par évaluation de la prolifération ou de la survie de cellules épidermiques en cultures, telles que des kératinocytes, ou par évaluation de l'activité antimicrobienne sur des bactéries en culture, telles que *Staphylococcus aureus* ou *Escherichia coli.*

De manière préférée, la détermination d'un état de la peau ou la caractérisation de l'efficacité d'un traitement cosmétique de la peau peuvent être effectuées par mesure de la variation de l'expression d'une séquence d'acides aminées, et de préférence représentée par une séquence choisie parmi SEQ ID NO: 16, SEQ ID NO: 17, SEQ ID NO: 18, SEQ ID NO : 19 ou SEQ ID NO : 20, un analogue ou un fragment de celle-ci.

Les procédés du brevet sont particulièrement avantageux dans la mesure où leur mise en oeuvre ne nécessite pas de recourir à une technique invasive. Un échantillon d'épiderme peut ainsi être obtenu par des techniques dites de « stripping » et directement analysé par une technique d'analyse conventionnelle connue de l'homme de l'art.

Ces strippings sont des surfaces collantes appliquées à la surface de l'épiderme comme le Blenderm® de 3M, le D'squam (adhésif commercial de CuDERM), la colle cyanoacrylate ou la méthode du « stripping » vernis. Grâce à ces « strippings », les cornéocytes adhérents et le contenu de leurs espaces intercellulaires peuvent être prélevés et soumis par la suite à une extraction permettant d'avoir accès au contenu protéique.

Le prélèvement d'un échantillon convenant à un procédé du brevet peut aussi être effectué de façon plus directe par « lavages » de la surface cutanée, au moyen par exemple d'accessoires de type turbine à ailette, de type cellule à spirale telle que décrit dans le brevet FR 2 667 778 associée à un circuit fluidique, ou simplement par ajout/prélèvement d'une goutte de tampon à la surface de la peau.

A titre indicatif, d'autres méthodes de prélèvement adaptées à la mise en oeuvre des procédésdu brevet peuvent être mentionnées, telles que des méthodes par grattage de la partie supérieure du *stratum corneum* au moyen d'un système bilames. Cette technique permet de recueillir des squames qui peuvent ensuite être directement analysés par différentes techniques pour déterminer les taux en minéraux, aminoacides ou lipides.

Avantageusement, un des marqueurs du brevet peut être mis en oeuvre à des fins de sélection préclinique, plus efficace et plus rigoureuse, d'individus, en vue d'évaluer l'efficacité de traitement ou d'actif cosmétique pour le soin de la peau.

Egalement avantageusement, un des marqueurs du brevet peut être mis en oeuvre à des fins de sélection préclinique, plus efficace et plus rigoureuse, d'individus, en vue d'évaluer l'efficacité de traitement ou d'actif cosmétique pour le soin de la peau sèche.

Egalement, un biomarqueur du brevet peut avantageusement être mis en oeuvre comme indiqué précédemment pour évaluer l'efficacité d'un agent actif, *in vitro*, *ex vivo* ou *in vivo* à l'égard d'un déficit d'hydratation de la peau, ou des peaux sèches ou des signes de sécheresse cutanée.

Egalement, un biomarqueur du brevet peut avantageusement être mis en oeuvre comme indiqué précédemment pour évaluer l'efficacité d'un agent actif, *in vitro*, *ex vivo* ou i*n vivo.*

Egalement, un biomarqueur du brevet peut être mis en oeuvre pour établir un conseil personnalisé d'un traitement cosmétique pour un individu en fonction de son profil d'expression de biomarqueurs cutanés.

Criblage

Selon un de ses aspects, le présent brevet concerne l'utilisation d'une séquence d'acides aminés, d'acides nucléiques telle que décrite dans le brevet, pour cribler ou dans un procédé de criblage, en particulier *in vitro* ou *ex vivo,* d'agents actifs ou de traitements physiques, convenant particulièrement au soin de la peau. Les agents actifs ou les traitements physiques criblés peuvent notamment convenir au soin de la peau âgée, et/ou à la prévention et/ou au traitement des signes cutanés du vieillissement, associé(e)(s) ou non à une sécheresse cutanée.

Il est également décrit l'utilisation d'une séquence d'acides aminés, d'acides nucléiques selon le brevet, pour cribler ou dans un procédé de criblage, en particulier *in vitro* ou *ex vivo,* d'agents actifs ou de traitements physiques, convenant particulièrement au soin de la peau et destinés à favoriser et/ou renforcer l'hydratation de la peau.

Egalement, les agents actifs ou les traitements physiques criblés peuvent notamment convenir au soin de la peau sèche, et/ou à la prévention et/ou au traitement des signes de sécheresse cutanée.

Une utilisation ou un procédé du brevet peuvent comprendre la comparaison d'une mesure de l'activité, de l'expression ou de la maturation d'une séquence d'acide aminés ou d'une séquence d'acides nucléiques selon le brevet à une mesure de référence.

Une mesure de référence peut être telle que définie précédemment.

En particulier une mesure de référence peut être une valeur quantitative ou qualitative relative à l'expression, la maturation ou l'activité desdites séquences déterminée dans un échantillon en l'absence d'agent actif ou de traitement physique testé.

Ainsi, une mesure de référence peut être obtenue en réitérant les étapes d'un procédé de l'invention, et notamment les étapes a), b) et c) d'un procédé de l'invention tel que défini précédemment, en l'absence de composés biologiques ou chimiques, ou de traitements physiques à tester.

Une comparaison de la mesure test à une mesure de référence, et une observation d'une déviation ou d'une absence de déviation entre les deux mesures, permet de tirer une information quant à l'effet de l'agent actif ou du traitement physique testé.

La détermination quantitative ou qualitative de l'expression, de la maturation ou de l'activité d'une séquence d'acides aminés ou d'une séquence d'acides nucléiques peut être effectuée par toute méthode connue de l'homme de l'art, et notamment comme décrit précédemment.

Selon un mode de réalisation, le criblage d'un agent actif ou d'un traitement physique susceptible de moduler l'activité d'une séquence d'acides aminées peut se faire par mesure de l'activité ou de l'expression d'une molécule cible appartenant aux voies de signalisation ou de métabolisme dans lesquelles peut être impliqué ladite d'une séquence d'acides aminées, tel que par exemple un système de gène reporteur.

Selon un mode de réalisation, un procédé selon le brevet peut être mis en oeuvre dans un système acellulaire, i.e. dans un système ne comprenant pas de cellules mais reproduisant des fonctions cellulaires, ou dans un échantillon cellulaire isolé.

Un procédé conforme au brevet peut être effectué sur un échantillon cellulaire isolé, un échantillon acellulaire, sur une séquence d'acides aminés isolée ou sur une séquence d'acides nucléiques isolée décrite par le brevet, obtenus par biopsie cutanée, à partir de cellules en culture, notamment à partir d'un modèle épidermique, ou d'un prélèvement de surface cutané non-invasif, notamment par adhésif (« tape-stripping ») de *stratum corneum* ou par simple lavage, comme décrit précédemment.

Avantageusement, à titre d'échantillon cellulaire convenant au brevet, on peut mentionner un échantillon de kératinocytes ou tout autre type cellulaire de la peau exprimant une séquence d'acides aminés décrite par le brevet.

De manière préférée, le criblage d'un agent actif ou d'un traitement physique peut être effectuée par mesure de la variation de l'expression, en présence et en absence de l'agent actif ou du traitement physique criblé, d'une séquence d'acides aminées selon le brevet, et de préférence représentée par une séquence choisie parmi SEQ ID NO: 16, SEQ ID NO: 17, ou SEQ ID NO: 18, un analogue ou un fragment de celle-ci.

Agent modulateur

On entend par « agent modulateur » ou « agent actif ou un traitement physique susceptible de moduler l'expression, la maturation ou l'activité d'une séquence d'acides aminées ou d'une séquence d'acides nucléiques », tout composé ou phénomène physique susceptible d'agir, directement ou indirectement, sur au moins une séquence d'acides aminées ou une séquence d'acides nucléiques conformes au brevet, ou sur un élément d'une voie de signalisation intra ou extra-cellulaire, ou d'une voie métabolique, ou de régulation de la transcription et/ou de la traduction impliquant ladite séquence d'acides aminées ou ladite séquence d'acides nucléiques.

On entend par « moduler », au regard d'un effet donné, l'action de stimuler ou d'inhiber cet effet.

Les agents actifs ou les traitements physiques issus d'un criblage selon le brevet peuvent être avantageusement utilisés à des fins cosmétiques, notamment au regard de la peau âgée ou des signes cutanés du vieillissement, associé(e)(s) ou non à une sécheresse cutanée. Il est également décrit que les agents actifs ou les traitements physiques issus d'un criblage selon le brevet peuvent être avantageusement utilisés à des fins cosmétiques, notamment au regard de l'hydratation de la peau, et en particulier de la peau sèche ou des signes de sècheresse cutanée.

Selon un mode de réalisation, un agent actif selon le brevet peut être un agent stimulateur de l'activité, de l'expression ou de la maturation d'une séquence d'acides aminées de l'invention.

En particulier, un agent stimulateur peut être choisi parmi choisi parmi un lysat de *Bifidobacterium sp.* et un mélange composé d'un lysat de *Bifidobacterium sp.* et de phytosphingosine-salicylate.

De manière préféré un lysat de *Bifidobacterium sp.* peut être un lysat de *Bifidobacterium longum*. Un lysat de *Bifidobacterium longum* convenant à une mise en oeuvre selon le brevet peut être le lysat enregistré sous le nom INCI : Bifidat ferment Lysate, sous le nom EINECS : *Bifidobacterium longum*, sous le N° EINECS : N° 306-168-4, et sous le N° CAS : N° 96507-89-0. Un tel lysat est notamment commercialisé sous la dénomination Repair Complex CLR® par la société K. RICHTER GmbH.

Un mélange composé d'un lysat de *Bifidobacterium sp.* et de phytosphingosine-salicylate selon le brevet peut comprendre un lysat de *Bifidobacterium longum* tel que défini précédemment et un dérivé de phytosphingosine-salicylate.

Un dérivé de phytosphingosine-salicylate convenant à la mise en oeuvre du brevet peut être le dérivé commercialisé par la société EVONICK GOLDSCHMIDT sous la dénomination Phytosphingosine SLC®.

Un mélange selon le brevet peut comprendre un lysat de *Bifidobacterium sp.* à raison de 10 % et un dérivé de phytosphingosine-salicylate à raison de 0,002 %.

A titre d'agent modulateur susceptible d'être criblé selon une utilisation ou un procédé conforme au brevet, on peut également mentionner les anticorps, ou les ARN interférents.

Compositions

Le présent brevet concerne également des compositions, notamment cosmétiques, comprenant dans un milieu physiologiquement ou cosmétiquement acceptable une quantité efficace d'au moins une séquence d'acides aminés décrite par le brevet, ou d'au moins une séquence d'acides nucléiques décrite par le brevet,.

Plus particulièrement, le brevet décrit une composition cosmétique comprenant un peptide représenté par une séquence d'acides aminés choisie parmi SEQ ID NO: 17 ou SEQ ID NO: 18, un analogue ou un fragment de celle-ci ou une séquence d'acides nucléiques codant pour un tel peptide.

L'invention se rapporte à une composition cosmétique comprenant un peptide isolé représenté par la séquence d'acides aminés SEQ ID NO: 18 ou une séquence d'acides nucléiques codant pour un tel peptide.

Au sens de la présente invention, on entend désigner par « milieu physiologiquement acceptable », un milieu convenant à l'administration d'une composition par voie topique sur la peau, le cuir chevelu, ou les lèvres, ou par voie orale ou par voie parentérale telle que la voie intradermique ou sous-cutanée.

Une composition selon le brevet peut contenir des adjuvants habituels dans le domaine considéré, tels que des gélifiants hydrophiles ou lipophiles, des additifs hydrophiles ou lipophiles, des conservateurs, des antioxydants, des solvants, des parfums, des charges, des filtres, des absorbeurs d'odeurs et des matières colorantes.

Les quantités des différents constituants des compositions selon le brevet sont celles classiquement utilisées dans les domaines considérés.

La quantité de séquence d'acides aminés, ou de séquence d'acides nucléiques décrite dans le brevet, encore dite « quantité efficace » est, bien entendu, fonction de la nature de l'actif et de l'effet recherché et peut donc varier dans une large mesure.

Pour donner un ordre de grandeur, une composition peut contenir une séquence d'acides aminés, ou une séquence d'acides nucléiques, en une quantité représentant de 0,00001 % à 50 % du poids total de la composition, en particulier en une quantité représentant de 0,001 % à 10 % du poids total de la composition, et plus particulièrement en une quantité représentant de 0,1 % à 1 % du poids total de la composition.

Selon un autre mode de réalisation, une composition cosmétique du brevet peut comprendre, en outre, au moins un agent actif cosmétique et/ou thérapeutique additionnel.

Actifs additionnels

Comme exemples d'agents actifs additionnels utilisables dans le cadre du présent brevet, il peut être fait mention d'huiles cosmétiques, telles que les huiles de silicone, les huiles végétales de type triglycérides, les huiles hydrocarbonées telles que l'huile de PARLEAM et les esters d'acides gras et d'alcool gras.

Il peut également être possible d'utiliser d'autres agents actifs permettant d'améliorer l'état de la peau et/ou de ses annexes, tels que des actifs hydratants ou humidifiants ou des agents actifs permettant d'améliorer la barrière lipidique naturelle, tels que des céramides, des sulfates de cholestérol et/ou des acides gras, et leurs mélanges.

Il peut encore être également possible d'utiliser d'autres agents actifs permettant d'améliorer l'état de la peau et/ou de ses annexes, tels que des actifs anti-âges.

Il peut également être possible d'utiliser des enzymes ayant une activité sur la peau et/ou de ses annexes, telles que des protéases, des lipases, des glucosidases, des amidases, des cérébrosidases et/ou des mélanases, et leurs mélanges.

Comme autres exemples d'agents actifs convenant à la mise en oeuvre du présent brevet figurent : des actifs analgésiques, des actifs anti-levures, des actifs antibactériens, des actifs antiparasitaires, des actifs antifongiques, des actifs antiviraux, des actifs anti-inflammatoires stéroïdiens, des actifs anesthésiques, des actifs antiprurigineux, des actifs kératolytiques, des actifs anti-radicaux libres, des actifs anti-séborrhéiques, des actifs antipelliculaires, des actifs anti-acnéiques, des actifs visant à prévenir le vieillissement de la peau et/ou à améliorer son état, des actifs anti-dermatites, des actifs anti-irritants, des actifs immuno-modulateurs, des actifs pour le traitement de la peau sèche, des actifs anti-transpirants, des actifs anti-psoriatiques, des actifs protecteurs contre les UV, des actifs antihistaminiques, des actifs cicatrisants, des actifs auto-bronzants, des antioxydants tels que le thé vert ou des fractions actives de celui-ci, la glycérine, la laponite, la caféine, des huiles essentielles aromatiques, des colorants, des actifs dépigmentants, des lipo-régulateurs, des actifs adoucissants, rafraîchissants, déodorants, insensibilisants, blanchissants, nourrissants, des actifs diminuant la différenciation et/ou la prolifération et/ou la pigmentation cutanée, et leurs mélanges.

A titre d'actifs additionnels susceptibles de convenir plus particulièrement à la mise en oeuvre du brevet, on peut mentionner également des microorganismes probiotiques, autres que ceux considérés dans le lysat précédemment décrit, des actifs prébiotiques, des actifs favorisant la synthèse de facteur de défense de la peau, des actifs permettant de rétablir l'équilibre différentiation/prolifération des cellules de l'épiderme, en particulier tels que des actifs de type retinol ou retinol-like, des actifs hydratants ou des actifs stimulant l'activité de protéase cutanée, notamment de la Cathépsine D.

Utilisation cosmétique

Le présent brevet concerne l'utilisation cosmétique d'une quantité efficace d'au moins une séquence d'acides aminés ou d'acides nucléiques décrite dans le brevet, à titre d'agent actif pour prévenir et/ou traiter la peau âgée et/ou les signes cutanés du vieillissement, associé(e)(s) ou non à une sécheresse cutanée.

Il est également décrit l'utilisation cosmétique d'une quantité efficace d'au moins une séquence d'acides aminés ou d'acides nucléiques décrite dans le brevet ou d'au moins un agent modulateur de l'activité, de l'expression ou de la maturation de ladite séquence d'acides aminés ou de ladite séquence d'acides nucléiques, et notamment un agent modulateur tel que défini précédemment, à titre d'agent actif pour favoriser et/ou renforcer l'hydratation de la peau.

Il est encore décrit qu'une utilisation selon le brevet peut être dédiée à prévenir et/ou traiter une peau sèche et/ou des signes de sécheresse cutanée.

Selon un autre aspect, la présente invention concerne un procédé cosmétique pour prévenir et/ou traiter une peau âgée et/ou des signes cutanés du vieillissement, associé(e)(s) ou non à une sécheresse cutanée chez un individu en ayant besoin, comprenant au moins une étape consistant à administrer audit individu, au moins une composition comprenant à titre d'agent actif (i) au moins une séquence d'acides aminés représentée par une séquence choisie parmi SEQ ID NO: 11, SEQ ID NO : 12, SEQ ID NO : 13 et SEQ ID NO : 16, ou (ii) au moins une séquence d'acides nucléiques représentée par une séquence choisie parmi SEQ ID NO: 1, SEQ ID NO : 2, SEQ ID NO : 3 et SEQ ID NO : 6.

Un procédé ou une utilisation du brevet permettent de prévenir et/ou traiter le vieillissement cutané, notamment chronologique et/ou photo-induit.

Un procédé ou une utilisation selon le brevet permettent de conférer et/ou de restaurer un aspect jeune à la peau, se manifestant par une peau lisse, élastique, tonique, ou ferme, ou un teint lumineux.

Un procédé ou une utilisation selon le brevet permettent de prévenir et/ou réduire la présence d'un microrelief marqué de la peau, en particulier au niveau de la commissure des lèvres et des pattes d'oies.

Un procédé ou une utilisation selon le brevet permettent de renforcer les propriétés barrières de la peau.

Dans le présente brevet, il est également décrit un procédé cosmétique, ou non thérapeutique, pour favoriser et/ou renforcer l'hydratation de la peau, et de préférence pour prévenir et/ou traiter une peau sèche et/ou des signes de sécheresse cutanée, chez un individu en ayant besoin, le procédé comprenant au moins une étape consistant à administrer audit individu, au moins une composition comprenant à titre d'agent actif (i) au moins une séquence d'acides aminés décrite par le brevet ou (ii) au moins une séquence d'acides nucléiques décrite par le brevet, ou (iii) au moins un agent modulateur de l'activité, de l'expression ou de la maturation desdites séquences décrites par le brevet, notamment tels que définis ci-avant.

Un procédé ou une utilisation selon le brevet permettent également de conférer et/ou de restaurer un aspect hydraté et sain à la peau, se manifestant par une peau lisse, élastique, tonique, ou ferme, ou un teint lumineux.

Un procédé ou une utilisation selon le brevet permettent également de prévenir et/ou de réduire la présence de squames, de rugosités, de crevasses, d'écailles et/ou de rides ou ridules associées à la peau sèche ou en déficit d'hydratation.

Un procédé ou une utilisation selon le brevet permettent également de renforcer les propriétés barrières de la peau.

De manière préférée, un procédé selon le brevet peut comprendre l'application par voie topique sur au moins une partie de la peau d'un individu en ayant besoin, en particulier sur la peau du visage et/ou du décolleté, d'au moins une couche d'une composition topique de l'invention.

Avantageusement, un procédé selon le brevet par voie topique peut comprendre l'application sur la peau, et notamment sur la peau du visage, d'une composition selon le brevet sous forme d'un masque.

Un procédé cosmétique par voie topique selon le brevet peut avantageusement comprendre l'application d'une composition selon le brevet, en association de manière simultanée, successive ou séparée dans le temps avec une composition cosmétique ou dermatologique additionnelle distincte de la composition selon le brevet et destinée au soin et/ou au maquillage de la peau.

Selon un autre mode de réalisation préféré, un procédé cosmétique selon le brevet peut être mis en oeuvre par voie orale, notamment par administration d'au moins une composition alimentaire ou diététique à visée cosmétique.

Selon un autre mode de réalisation préféré, un procédé cosmétique selon le brevet peut être mis en oeuvre par voie parentérale. La mise en oeuvre par voie parentérale d'un procédé cosmétique selon le brevet est effectuée à l'exclusion de toute intervention chirurgicale et ne vise qu'à exercer un traitement de surface de la peau à des fins esthétiques.

Ainsi, un procédé cosmétique selon le brevet par voie parentérale est mis en oeuvre par toute technique d'injection ou dispositif convenant à une injection intra-épidermique et/ou intradermique et/ou sous-cutanée.

Une telle administration peut être effectuée, par exemple, par mésothérapie.

Un procédé cosmétique par voie parentérale ne se traduit donc que par une effraction superficielle de la peau et sort donc de tout cadre médical ou thérapeutique.

Pour la voie parentérale, on pourra alternativement privilégier l'administration par patch à visée systémique.

Un procédé cosmétique selon le brevet peut être mis en oeuvre de façon journalière, par exemple à raison d'une administration unique par jour, ou d'une administration fractionnée en deux ou trois fois par jour, par exemple une fois le matin et une fois le soir.

Un procédé cosmétique selon le brevet peut être mis en oeuvre sur une période de temps variant d'une semaine à plusieurs semaines, voire plusieurs mois, cette période pouvant par ailleurs être répétée après des périodes de non traitement pendant plusieurs mois, voire plusieurs années.

A titre d'exemple, de procédé cosmétique selon le brevet on peut prévoir une administration d'une composition décrite par le brevet, par exemple, à raison de 1, 2 ou 3 fois par jour, ou plus, et généralement sur une durée prolongé d'au moins 4 semaines, voire 4 à 15 semaines, avec le cas échéant une ou plusieurs périodes d'interruption.

<u>Peau reconstruite</u>

Selon un autre aspect, le présent brevet concerne l'utilisation d'une quantité efficace d'au moins une séquence d'acides aminés décrite par le brevet, ou d'au moins une séquence d'acides nucléiques décrite par le brevet, pour préparer un modèle cellulaire épithélial pluristratifié, de préférence un modèle de peau reconstruite.

L'intérêt de développer des modèles organotypiques les plus proches de conditions *in-vivo* est grand pour l'évaluation sécurité et efficacité des actifs et formules à applications cosmétiques et dermatologiques.

L'utilisation d'au moins une séquence d'acides aminés décrite par le brevet, ou d'au moins une séquence d'acides nucléiques décrite par le brevet, ou d'au moins un agent modulateur décrit par le brevet dans un milieu de culture est susceptible d'améliorer la qualité des modèles développés.

Un modèle de peau reconstruite selon le brevet peut être utile pour mimer une peau âgée, éventuellement associée à une sècheresse cutanée, ou les conditions d'une restauration de l'homéostasie d'une peau âgée, éventuellement associée à une sécheresse cutanée.

Selon un autre aspect, le présent brevet concerne un procédé de préparation d'un modèle cellulaire épithélial pluristratifié isolé, et de préférence une peau reconstruite isolée comprenant, au moins l'étape de mettre en contact au moins une quantité efficace d'au moins une séquence d'acides aminés, ou d'au moins une séquence d'acides nucléiques, avec des cellules susceptibles de générer une peau reconstruite isolée, et notamment des kératinocytes.

Un modèle de peau reconstruite peut comprendre différents types cellulaires, tels des kératinocytes, des fibroblastes, des cellules de Langerhans et des mélanocytes. Les cellules de type fibroblastes peuvent être irradiées ou non.

De tels modèles et leur préparation sont connus de l'homme de l'art.

Selon encore un autre aspect, le présent brevet décrit également un procédé de préparation d'un modèle cellulaire épithélial pluristratifié, de préférence un modèle de peau reconstruite, comprenant au moins une étape de mise en culture de cellules d'au moins un type cellulaire dudit modèle, lesdites cellules ayant été génétiquement modifiées pour supprimer l'expression d'une séquence d'acides aminés ou d'une séquence d'acide nucléiques décrite par le brevet.

L'obtention de cellules génétiquement modifiées pour supprimer l'expression d'une séquence d'acides aminés ou d'une séquence d'acide nucléiques décrite par le brevet, cellules dites « knock-out », peut être réalisée par toute méthode connue de l'homme de l'art.

A titre d'exemple, de telles cellules peuvent être obtenues par transfection et recombinaison homologue d'un fragment d'acide nucléique venant s'insérer dans ou prendre la place du gène exprimant la séquence d'acides aminés dont l'expression est à supprimer.

Egalement, il peut être possible de supprimer l'expression d'un gène donné par transfection dans la cellule d'une séquence d'acide nucléique codant pour un ARN interférant spécifique de l'ARNm issu du gène dont l'expression est à supprimer.

## Légendes des figures

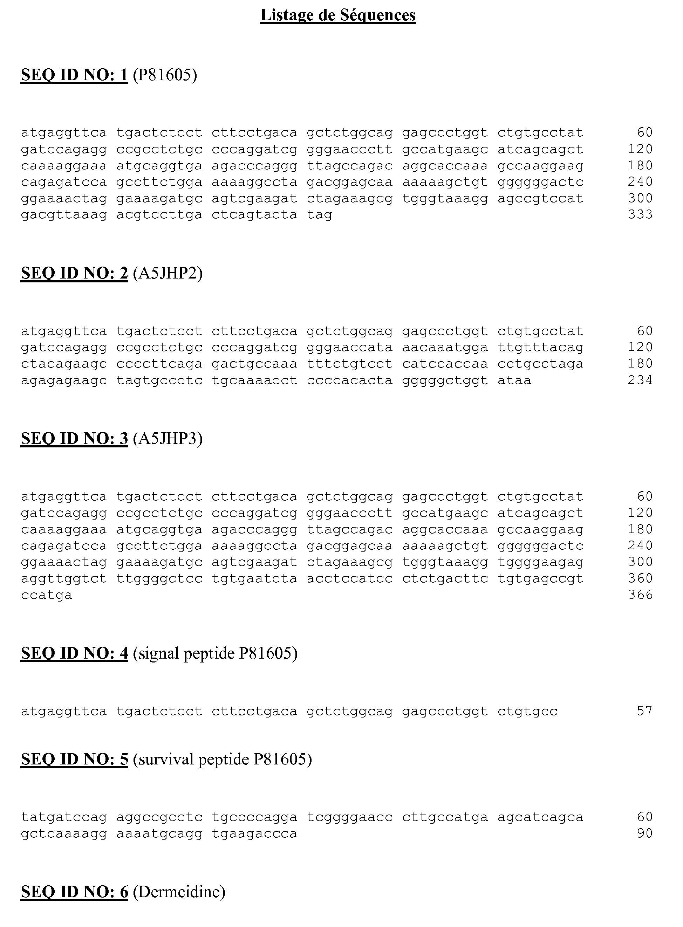

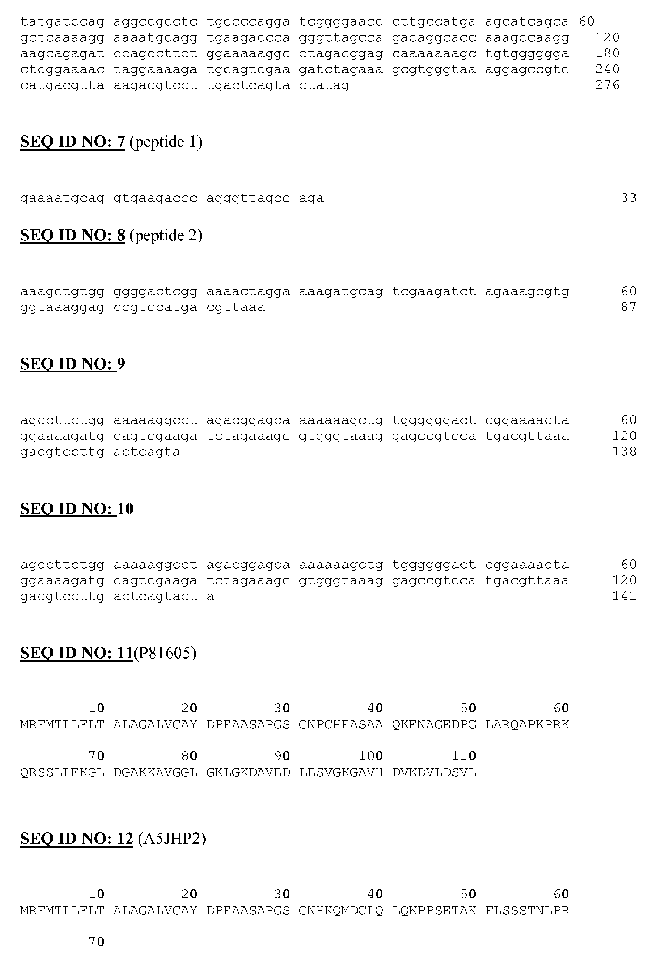

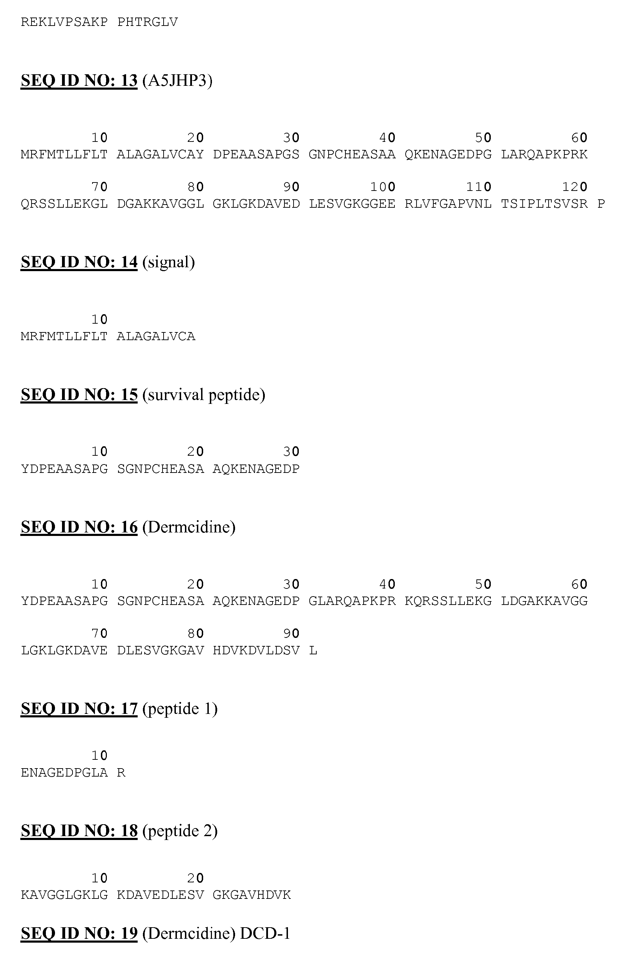

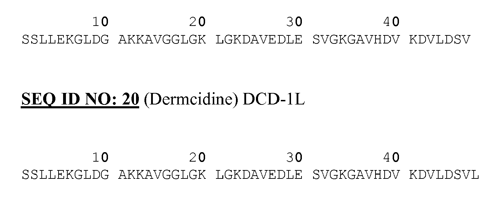

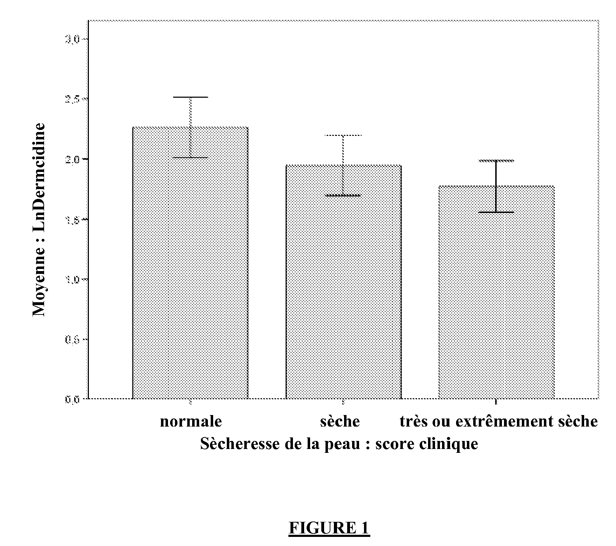

**Figure 1 :** représente la variation de l'expression de la Dermcidine (moyenne ± sem) mesurée par Western-Blot en fonction du degré d'hydratation de la peau.

Au sens du présent brevet, « un » doit se comprendre, sauf indication contraire, au sens de « au moins un ».

Les exemples et figures ci-après sont présentés à titre illustratif et non limitatif.

## EXEMPLES

### Exemple 1

*Expression de la Dermcidine, dans la peau*

1- Matériels et méthodes

a- Protocole iTRAQ

La détection de l'expression de la Dermcidine (DCD) a été réalisée au moyen de la méthode iTRAQ (Isobaric Tagging Reagents for Quantitative Proteomic Analysis) développée par la société Applied Biosystems. Cette méthode permet la quantification de peptides préalablement marqués par un Tag isobarique. Il existe 4 tags différents et on peut comparer jusqu'à 4 types d'échantillons distincts dans une même analyse LC/MS-MS.

Le principe de cette méthode est le suivant :

- Digestion d'extraits protéiques et marquage :

Dans un premier temps, chaque extrait protéique est digéré par une enzyme protéolytique : la trypsine.

Dans un second temps, chaque mélange peptidique obtenu est marqué avec un Tag isobarique d'une masse totale 145, comprenant un groupe « reporter » de masse 114, 115, 116 ou 117, et un groupe « balance » de masse 31, 30, 29 ou 28, liés entre eux par un site de fragmentation MS.

Le marquage est obtenu par réaction du Peptide Reactive Group de chaque tag avec les amines primaires des peptides (extrémité N-terminale des peptides ou la chaîne latérale des lysines ou des tyrosines.

- Analyse LC/MS-MS et quantification :

Après l'étape de marquage, les échantillons sont mis en commun et analysés par spectrométrie de masse. Un peptide donné, présent dans plusieurs échantillons, aura une masse parente identique mais son spectre de fragmentation MS/MS va générer la masse du groupe rapporteur caractéristique de chacun des réactifs iTRAQ. La comparaison de l'intensité de chaque ion rapporteur (114, 115, 116 ou 117) permet alors la quantification du peptide.

La méthode iTRAQ est plus précisément décrite par Zieske (J. Exp. Bot., 2006, 57 :1501) ou Wiese et al. (Proteomics, 2007, 7 :340).

b- Prélèvement et traitement des échantillons cutanés

Des prélèvements de type stripping-vernis de stratum corneum d'une surface de 90 $cm^2$, sont obtenus selon le protocole décrit par Mehul et al., J. Biol. Chem., 2000, 275(17):12841-7, au niveau de la face externe de la jambe. Deux groupes de sujets sont prélevés :

1) Groupe « Peau Jeune » : 18-40 ans, 27 sujets masculins ;
2) Groupe « Peau Agée » : supérieur à 60 ans, 35 sujets masculins.

Ces prélèvements de peau sont ensuite analysés par protéomique au moyen de la technique dite de « marquage isobarique », précédemment décrite, en utilisant le kit iTRAQ Reagents Multiplex kit (4352135), d'Applied Biosystems, conformément aux instructions du fabricant.

2- Résultats

Parmi les protéines identifiées, les peptides représentés par les séquences SEQ ID NO: 17 et SEQ ID NO: 18 issus de la Dermcidine (N° accession P81605 - Réf.: Swissprot/Uniprot) sont détectés et quantifiés dans quatre expériences.

Les résultats de quantification de la Dermcidine sont donnés dans le tableau suivant :

| **Protéine** | **Expérience N° Ratio 116/114 âgé / jeune** | | | | | |
|---|---|---|---|---|---|---|
| | **1** | **2** | **3** | **4** | **Moyenne** | **Ecart-type** |
| Dermcidine | 0,3937 | 0,2465 | 0,4356 | 0,4209 | **0,37** | **0,09** |

Ces résultats montrent que le ratio iTRAQ moyen âgé/jeune est égal à 0,37 $\pm$ 0,09. Cette mesure traduit une forte expression de la Dermcidine dans les peaux du groupe « Peau jeune », et une forte diminution de son expression dans les peaux du groupe « Peau âgée ».

La Dermcidine se révèle donc être un biomarqueur intéressant et spécifique de la peau, et plus particulièrement d'un état âgé de la peau, associé ou non à une sécheresse cutanée.

L'expression de l'Apolipoprotéine D et de la chaîne lourde de la Cathepsine D a été mesurée à titre de données comparatives.

Les résultats obtenus sont exprimés dans le tableau suivant.

| **Protéine** | **Expérience N° Ratio 116/114 âgé / jeune** | | | | | |
|---|---|---|---|---|---|---|
| | **1** | **2** | **3** | **4** | **Moyenne** | **Ecart-type** |
| Apolipoprotéine D | 0,7975 | 0,7455 | 0,8205 | 0,9062 | **0,82** | **0,07** |
| Chaîne lourde de la Cathepsine D | 0,9580 | 1,1068 | 0,9595 | 0,9537 | **0,99** | **0,07** |

En comparaison de la Dermcidine, l'Apolipoprotéine D et la chaîne lourde de la Cathépsine D ne sont que très légèrement sous-exprimées dans le groupe « Peau âgée » par rapport au groupe « Peau jeune ».

Cette étude valide ainsi l'utilisation de la Dermcidine à titre de biomarqueur d'exception de la peau, et plus particulièrement de la peau âgée, associée ou non à une sécheresse cutanée.

**Exemple 2**

*Expression de la Dermcidine dans la peau*

1- Matériels et méthodes

a- Protocole Western-Blot

La détection de l'expression de la Dermcidine (DCD) a été réalisée au moyen d'une méthode de Western-Blot.

Le principe de cette méthode est le suivant : les protéines sont séparées par migration électrophorétique sur un gel Critérion SDS-PAGE 10-20 % (Bio-Rad). Après un transfert semi-sec sur membrane PVDF, les protéines sont incubées dans une solution de blocage (TBS+ 0.1 %Tween+ 1 % lait) puis incubées avec l'anticorps primaire dirigé contre la protéine d'intérêt pendant une nuit à 4 °C. Une seconde incubation avec l'anticorps secondaire (couplé à une peroxydase) dirigé contre l'anticorps primaire est effectuée pendant une heure à température ambiante.

Selon le degré de sensibilité, différents kits sont utilisés pour la révélation : ECL, ECL+ ou ECL Advance (Amersham). L'image est acquise au FluorSmax (Biorad) et les bandes quantifiées à l'aide d'un logiciel Quantity-One (Biorad)

| Protéine | Nature échantillons | Anticorps I | Dilution | Anticorps II | Détection | Poids mol. calculé |
|---|---|---|---|---|---|---|
| Dermcidine | Dénaturé | Sc-27466/poly | 1/100 | IgG anti-Chèvre-HRP | ECL+ 60sec | 9kDa |

b- Prélèvement et traitement des échantillons cutanés

120 individus de sexe féminin, âgés de 18 à 70 ans, ont été sélectionnés pour cette étude.

Quatre zones par jambe ont été délimitées (jambe gauche externe/interne, jambe droite externe/interne). Les prélèvements sont réalisés par Stripping Vernis (site du bas de la jambe) selon le protocole décrit par Mehul et al., (J. Biol. Chem., 2000, 275(17):12841-7).

Pour cette étude, l'analyse est effectuée sur une zone : jambe gauche externe.

Les échantillons issus des strippings vernis ont été utilisés pour l'analyse en Western Blot. Avant la réalisation des prélèvements, les types de peaux ont été caractérisés par un scorage clinique.

Le scorage clinique est réalisé par un dermatologue selon l'« Atlas de la peau sèche sur les jambes » afin d'identifier les différents types de peaux selon les degrés ci-dessous :

0 : peau normale, relief cutané régulier, aspect lisse
1 : peau légèrement sèche, relief cutané strié, aspect rêche
2 : peau sèche, relief cutané strié et quelques squames, aspect rêche
3 : peau très sèche, squames nombreuses et quelques écailles, aspect rugueux
4 : peau extrêmement sèche, écailles nombreuses, aspect rugueux

*Préparation des poudres acétoniques*

Chaque stripping vernis de 75cm$^2$ est plongé dans 75ml d'acétone. Le vernis se dissout et les cornéocytes sont en suspension. Le mélange est filtré sur pompe à vide sur membrane de nylon de 40µm pour recueillir les cornéocytes. Deux lavages successifs sont effectués avec le même volume d'acétone. On obtient des poudres acétoniques de *stratum corneum* sous forme sèche.

*Extraction des échantillons*

Deux types d'extraction sont réalisés de manière successive : extraction native puis dénaturante.

Extraction native

Les poudres acétoniques sont pesées et mises dans des tubes Eppendorf de 1,5 ml (Trefflab cat.No.96.07246.9.01). 100 µl de tampon PBS 0,1 % triton X100 sont ajoutées pour 1 mg de poudre acétonique et le mélange est laissé une heure en contact dans la glace. Puis il est broyé pendant 30 secondes dans la glace. Le milieu est ensuite récupéré dans des colonnes Millipore Ultraree de 0,45 µm puis centrifugé pendant 5 min à 10000 g à 4 °C : le surnageant est alors recueilli. Un dosage de protéines est réalisé selon la technique du BCA (kit BCA Pierce). Les échantillons sont ajustés à une concentration de 0.15 mg/ml en tampon Laemmli 4X et conservés à -20 °C en attendant l'analyse.

Extraction dénaturante

Elle est réalisée à partir du culot de l'extrait natif. Dans des tubes Eppendorf de 1,5 ml (Trefflab cat.No.96.07246.9.01) 100µl de tampon Laemmli 1X est ajouté au culot pour 1 mg de poudre acétonique. Le mélange est bouilli pendant 10 min à 90 °C puis broyé pendant 20 secondes et centrifugé 10 min à 10000 g/min. Le surnageant est recueilli. Un dosage de protéines est réalisé selon la technique de Bradford. Les échantillons sont ajustés à 1 mg/ml et conservés à -20 °C en attendant l'analyse.

Ces prélèvements de peau sont ensuite analysés par Western-Blot comme précédemment décrit. Les résultats ont été obtenus à partir des extraits dénaturés.

*Analyses Statistique*

L'effet de la sécheresse cutanée (score clinique) sur le niveau d'expression des bio-marqueurs a été analysé à la fois graphiquement en représentant les valeurs moyennes avec leur intervalle de confiance en fonction des classes d'âge ou du score clinique de sécheresse cutanée, et à l'aide d'un modèle de régression multiple avec les paramètres âge et score de sécheresse cutanée en covariables.

La significativité des coefficients (test de Student) associés aux covariables permet de tester l'association entre le bio-marqueur étudié avec l'âge et le score de sécheresse. La prise en compte des deux paramètres dans le modèle permet d'estimer l'effet d'un des paramètres tout en ajustant sur l'effet du second (toute chose égale par ailleurs).

Le niveau de significativité des tests est fixé à 5 % dans une approche bilatérale non ajustée. Les analyses ont été effectuées à l'aide du logiciel SPPSS version 17. Les niveaux d'expression des bio-marqueurs ont été préalablement transformés (racine carrée ou Log10) lorsque cela était utile pour à la fois symétriser leur distribution et stabiliser leur variance.

2- <u>Résultats</u>

Les résultats obtenus, illustrés par la Figure 1, montrent clairement une diminution de l'expression de la Dermcidine corrélée avec une augmentation du degré de déshydratation, ou de sécheresse, de l'épiderme.

L'analyse statistique confirme une diminution significative de l'expression de la Dermcidine dans les peaux sèches par rapport aux peaux normales (p=0.046).

Cette étude valide ainsi l'utilisation de la Dermcidine à titre de biomarqueur d'exception de l'hydratation de la peau, et plus particulièrement de la peau sèche.

**<u>Exemple 3</u>**

*Effet d'un lysat de Bifidobacterium sp. sur l'expression de la Dermcidine*

1- <u>Matériels et méthodes</u>

Les prélèvements d'échantillons de peaux, et les mesures de l'expression de la Dermcidine sont réalisés comme indiqué à l'exemple 1.

Les volontaires sont des sujets féminins âgés de 30 à 65 ans. Ils ont été combinés et répartis en deux sous-groupes selon le traitement administré, un groupe « Placebo », et un groupe « Traité ».

Le groupe « Traité » reçoit par voie topique, sur la jambe, à raison de 2 fois par jour, matin et soir, pendant 56 jours, soit 2 mois, une composition comprenant 10 % de Repair Complex® dans une formule support correspondant à une émulsion huile dans eau déminéralisée Arlacel/Myrj contenant 5 % de Parleam, 15 % de cyclopentasiloxane, 3 % de glycérine et 2 % de vaseline.

Le Repair Complex® comprend un lysat de *Bifidobacterium longum* enregistré sous le nom INCI : Bifida ferment Lysate, sous le nom EINECS : *Bifidobacterium longum*, sous le N° EINECS : N° 306-168-4, et sous le N° CAS : N° 96507-89-0. Ce lysat est commercialisé sous la dénomination Repair Complex CLR® par la société K. RICHTER GmbH.

Le groupe « Placebo » reçoit en revanche uniquement la formule support.

Pour chaque condition sujets « Traité » et « Placebo », une moyenne des ratios J56/J0 est obtenue, permettant ainsi d'évaluer l'effet du traitement.

Les ratios J56/J0 sont calculés selon la formule suivante :

$$\% \text{ effet du traitement} = 100 * \frac{\text{Ratio J56/J0 « Traité » - Ratio J56/J0 « Placebo »}}{\text{ratio J56/J0 « Placebo »}}$$

2- <u>Résultats</u>

Les données brutes obtenues à l'issue de l'analyse sont les suivantes :

| Expérience 1 | |
|---|---|
| Ratio J56/J0 Placebo | Ratio J56/J0 Traité |
| 2,70 | 3,10 |
| **Expérience 2** | |
| Ratio J56/J0 Placebo | Ratio J56/J0 Traité |
| 1,00 | 1,62 |

La valeur moyenne du ratio J56/J0 « Placebo » est de 1,85, alors que la valeur moyenne du ratio J56/J0 « Traité » est de 2,36.

Le traitement par le Repair Complex® se traduit par une augmentation du signal de 27 % par rapport au placebo.

Ainsi l'administration, par voie topique, d'un lysat de *Bifidobacterium longum* accroît l'expression de la Dermcidine d'environ 30 % après 2 mois de traitement.

L'administration, par voie topique, d'un lysat de *Bifidobacterium sp.* permet donc de restaurer un niveau d'expression de la Dermcidine au niveau de la peau, et notamment de la peau âgée, associée ou non à une sécheresse cutanée, propice à renforcer son action de protection de la peau, à renforcer les propriétés barrières de la peau, et à réduire les signes cutanés du vieillissement, associés ou non à une sécheresse cutanée.

L'administration, par voie topique, d'un lysat de *Bifidobacterium sp.* permet également de restaurer un niveau d'expression de la Dermcidine au niveau de la peau sèche, propice à renforcer son action de protection de la peau, à renforcer les propriétés barrières de la peau, et à réduire les signes de sècheresse cutanée.

Cette étude valide en outre l'utilisation de la Dermcidine à titre de biomarqueur pour évaluer l'efficacité d'un traitement cosmétique de la peau âgée, ainsi qu'à titre d'outil de criblage de nouveaux agents actifs destinés au traitement de la peau âgée, associée ou non à une sécheresse cutanée.

**Exemple 4**

*Effet d'un mélange lysat de Bifidobacterium longum et de phytosphingosine salicylate sur l'expression de la Dermcidine*

1- Matériels et méthodes

Les prélèvements d'échantillons de peaux, et les mesures de l'expression de la Dermcidine sont réalisés comme indiqué à l'exemple 1.

Les volontaires sont des sujets féminins âgés de 40 à 45 ans. Chaque sujet est son propre témoin, les deux bras sont prélevés mais un seul est traité avec le produit testé et le second bras reçoit un placebo.

Le bras « Traité » reçoit par voie topique, à raison de 2 fois par jour, matin et soir, pendant 56 jours, soit 2 mois, une composition comprenant 10 % de Repair Complex® et 0,002 % de Phytosphingosine-SLC dans une formule support correspondant à une émulsion huile dans eau déminéralisée Arlacel/Myrj contenant 5 % de Parleam, 15 % de cyclopentasiloxane, 3 % de glycérine et 2 % de vaseline.

Le Repair Complex® comprend un *Bifidobacterium longum* enregistré sous le nom INCI : Bifida ferment Lysate, sous le nom EINECS : Bifidobacterium longum, sous le N° EINECS : N° 306-168-4, et sous le N° CAS : N° 96507-89-0. Ce lysat est commercialisé sous la dénomination Repair Complex CLR® par la société K. RICHTER GmbH.

Le dérivé phytosphingosine-salicylate est celui commercialisé par la société EVONICK GOLDSCHMIDT sous la dénomination Phytosphingosine SLC®.

Le bras « Placebo » reçoit uniquement la formule support.

Pour chaque condition « Traité » et « Placebo », une moyenne des ratios J56/J0 est obtenue comme indiqué précédemment, permettant ainsi d'évaluer l'effet du traitement.

2- Résultats

Les données brutes obtenues à l'issue de l'expérience sont les suivantes :

| Expérience n 1 | |
|---|---|
| Ratio J56/J0 Placebo | Ratio J56/J0 Traité |
| 0.75 | 1.66 |
| **Expérience n°2** | |
| Ratio J56/J0 Placebo | Ratio J56/J0 Traité |
| 0.45 | 0.81 |

La valeur moyenne du ratio J56/J0 « Placebo » est de 0,6, alors que la valeur moyenne du ratio J56/J0 « Traité » est de 1,235.

L'effet traitement est égal à 105%, et traduit un doublement de l'expression de la Dermcidine par rapport au « Placebo ».

Ainsi l'administration, par voie topique, d'un mélange comprenant un lysat de *Bifidobacterium longum* et de la phytosphingosine-salicylate accroît l'expression de la Dermcidine de plus de 100 %.

L'administration, par voie topique, d'un mélange comprenant un lysat de *Bifidobacterium longum* et de la phytosphingosine-salicylate permet donc de restaurer un niveau d'expression de la Dermcidine au niveau de la peau, et notamment de la peau âgée, associée ou non à une sécheresse cutanée, propice à renforcer son action de protection de la peau, à renforcer les propriétés barrières de la peau, et à réduire les signes cutanés du vieillissement, associés ou non à une sécheresse cutanée. L'administration, par voie topique, d'un mélange comprenant un lysat de *Bifidobacterium longum* et de la phytosphingosine-salicylate permet également de restaurer un niveau d'expression de la Dermcidine au niveau de la peau, et notamment de la peau sèche, propice à renforcer son action de protection de la peau, à renforcer les propriétés barrières de la peau, et à réduire les signes de sècheresse cutanée.

## BIBLIOGRAPHIE

Anderson, R. K. and W. L. Kenney (1987). « Effect of age on heat-activated sweat gland density and flow during exercise in dry heat » J. Appl. Physiol. 63(3): 1089-1094.

Baechle, D., T. Flad, et al. (2006). « Cathepsin D is present in human eccrine sweat and involved in the postsecretory processing of the antimicrobial peptide DCD-1L » J. Biol. Chem. 281(9):5406-5415.

Cunningham, T. J., L. Hodge et al. (1998) «Identification of a survival-promoting peptide in medium conditioned by oxidatively stressed cell lines of nervous system origin » J. Neurosci 18(18):7047-7060.

Flad, T., R. Bogumil et al. (2002) « Detection of dermcidin-derived peptides in sweat by ProteinChip Technology. » J. Immunol. Methods 270(1): 53-62.

Kenney, W. L. and S. R. Fowler (1988) « Methylcholine-activated eccrine sweat gland density and output as a function of age » J. Appl. Physiol. 65(3):1082-1086.

Kyte et al., (1982), J. Mol. Biol., 157: 105.

Mehul B. et al., (2000) « Identification and Cloning of a New Calmodulin-like Protein from Human Epidermis » J. Biol. Chem. 275(17):12841-12847

Moreira, D. F, B. E. Strauss et al. (2008) « Genes up- and down-regulated by dermcidin in breast cancer: a microarray analysis » Genet. Mol. Res. 7(3): 925-932.

Niyonsaba, F. A. Suzuki et al. (2009) « The human antimicrobial peptide dermcidin activates normal human keratinocytes » Br. J. Dermatol. 160(2): 243-249.

Rieg, S.C., Garbe et al. (2004) « Dermcidin is constitutively produced by eccrine sweat gland and is not induced in epidermal cells under inflammatory skin conditions » Br. J. Dermatol. 151(3): 534-539.

Rieg S., II Steffen et al. (2005) « Deficiency of dermcidin-derived antimicrobial peptides in sweat of patients with atopic dermatitis correlates with an impaired innate defense of human skin in vivo » J. Immunol. 174(12):8003-8010.

Sakurada K., T. Akutsu, et al., (2009) « Detection of dermcidin for sweat identification by real-time RT-PCR and ELISA » Forensic. Sci. Int. 2010 Jan. 30;194(1-3):80-4. Epub 2009 Nov. 13.

Sambrook et al., 1989, Vol. I-III, Coldspring Harbor Laboratory, Coldspring Harbor Press, NY.

Schittek, B, R. Hipfel et al. (2001) : « Dermcidin: a novel human antibiotic peptide secreted by sweat glands » Nat. Immunol. 2(12): 1133-11137.

Watchorn, T. M. N. Dowidar et al. (2005) « The chachectic mediator proteolysis inducing factor activates NF-kappaB and STAT3 in human Kupffer cells and monocytes » Int. J. Oncol. 27(4): 1105-1111.

Wiese et al., (2007) « Protein labeling by iTRAQ: A new tool for quantitative mass spectrometry in proteome research » Proteomics 7(3):340-350
Zieske et al. (2006) « A perspective on the use of iTRAQ reagent technology for protein complex and profiling studies » J. Exp. Bot. 57(7):1051-1058.

## Revendications

1. Utilisation (i) d'au moins une séquence d'acides aminés codée par une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10 ou (ii) de ladite séquence d'acides nucléiques, à titre de biomarqueur d'un état de la peau âgée et/ou des signes cutanés du vieillissement, associé(e)(s) ou non à une sécheresse cutanée.

2. Utilisation selon la revendication 1, dans laquelle ladite séquence d'acides aminés est représentée par une séquence choisie parmi SEQ ID NO: 11 à SEQ ID NO: 20, et de préférence est choisie parmi SEQ ID NO: 16, SEQ ID NO: 17, SEQ ID NO: 18, SEQ ID NO : 19, SEQ ID NO: 20.

3. Utilisation (i) d'au moins une séquence d'acides aminés représentée par une séquence choisie parmi SEQ ID NO: 11, SEQ ID NO : 12, SEQ ID NO : 13 et SEQ ID NO : 16, ou (ii) d'au moins une séquence d'acides nucléiques représentée par une séquence choisie parmi SEQ ID NO: 1, SEQ ID NO : 2, SEQ ID NO : 3 et SEQ ID NO : 6, pour cribler des agents actifs ou des traitements physiques pour le soin de la peau âgée et/ou la prévention et/ou le traitement des signes cutanés du vieillissement, associé(e)s ou non à une sécheresse cutanée, susceptibles de moduler l'activité, l'expression ou la maturation de ladite séquence d'acides aminés ou de ladite séquence d'acides nucléiques.

**4.** Utilisation (i) d'au moins une séquence d'acides aminés représentée par une séquence choisie parmi SEQ ID NO: 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20 , ou (ii) d'au moins une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, pour caractériser l'efficacité d'un traitement cosmétique de la peau âgée et/ou les signes cutanés du vieillissement, associé(e)(s) ou non à une sécheresse cutanée.

**5.** Utilisation cosmétique d'une quantité efficace (i) d'au moins une séquence d'acides aminés représentée par une séquence choisie parmi SEQ ID NO: 11, SEQ ID NO : 12, SEQ ID NO : 13 et SEQ ID NO : 16, ou (ii) d'au moins une séquence d'acides nucléiques représentée par une séquence choisie parmi SEQ ID NO: 1, SEQ ID NO : 2, SEQ ID NO : 3 et SEQ ID NO : 6, à titre d'agent actif pour prévenir et/ou traiter la peau âgée et/ou les signes cutanées du vieillissement, associé(e)(s) ou non à une sécheresse cutanée.

**6.** Utilisation d'une quantité efficace (i) d'au moins une séquence d'acides aminés représentée par une séquence choisie parmi SEQ ID NO: 11, SEQ ID NO : 12, SEQ ID NO : 13 et SEQ ID NO : 16, ou (ii) d'au moins une séquence d'acides nucléiques représentée par une séquence choisie parmi SEQ ID NO: 1, SEQ ID NO : 2, SEQ ID NO : 3 et SEQ ID NO : 6, pour préparer un modèle cellulaire épithélial pluristratifié, de préférence un modèle de peau reconstruite, mimant une peau âgée, éventuellement associée à une sécheresse cutanée.

**7.** Procédé *in vitro* ou *ex vivo* pour caractériser un état de la peau âgée et/ou les signes cutanées du vieillissement, associé(e)(s) ou non à une sécheresse cutanée comprenant au moins les étapes consistant à :

a) effectuer, dans un échantillon isolé d'une peau, une mesure qualitative ou quantitative de l'expression, de la maturation ou de l'activité d'au moins une séquence d'acides aminés représentée par une séquence choisie parmi SEQ ID NO: 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20 ou d'au moins une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, et
b) comparer ladite mesure effectuée à l'étape a) à une mesure de référence.

**8.** Procédé de criblage *in vitro* ou *ex vivo* d'agents actifs ou de traitements physiques pour le soin de la peau âgée et/ou la prévention et/ou le traitement des signes cutanés du vieillissement, associé(e)s ou non à une sécheresse cutanée, susceptibles de moduler l'activité, l'expression ou la maturation d'une séquence d'acides aminés représentée par une séquence choisie parmi SEQ ID NO: 11, SEQ ID NO : 12, SEQ ID NO : 13 et SEQ ID NO : 16, ou d'une séquence d'acides nucléiques représentée par une séquence choisie parmi SEQ ID NO: 1, SEQ ID NO : 2, SEQ ID NO : 3 et SEQ ID NO : 6, comprenant au moins les étapes consistant à :

a) disposer ladite séquence d'acides aminés ou ladite séquence d'acides nucléiques dans des conditions propices à l'activité, l'expression ou la maturation desdites séquences,
b) mettre en contact ladite séquence d'acides aminés ou ladite séquence d'acides nucléiques avec au moins un agent actif à tester, ou exposer ladite séquence d'acides aminés ou ladite séquence d'acides nucléiques avec un traitement physique à tester,
c) effectuer une mesure qualitative ou quantitative de l'expression, de la maturation ou de l'activité de ladite séquence d'acides aminés ou de ladite séquence d'acides nucléiques, et
d) comparer ladite mesure à une mesure de référence, et
e) observer une différence entre ladite mesure et la mesure de référence pour caractériser l'agent actif ou le traitement comme étant utile pour prévenir et/ou traiter la peau âgée et/ou les signes cutanés du vieillissement, associé(e)(s) ou non à une sécheresse cutanée.

**9.** Procédé cosmétique pour prévenir et/ou traiter une peau âgée et/ou des signes cutanés du vieillissement, associé(e)(s) ou non à une sécheresse cutanée chez un individu en ayant besoin, comprenant au moins une étape consistant à administrer audit individu, au moins une composition comprenant à titre d'agent actif (i) au moins une séquence d'acides aminés représentée par une séquence choisie parmi SEQ ID NO: 11, SEQ ID NO : 12, SEQ ID NO : 13 et SEQ ID NO : 16, ou (ii) au moins une séquence d'acides nucléiques représentée par une séquence choisie parmi SEQ ID NO: 1, SEQ ID NO : 2, SET ID NO : 3 et SEQ ID NO : 6.

**10.** Procédé cosmétique, *in vitro* ou *ex vivo*, pour caractériser l'efficacité d'un traitement cosmétique de la peau âgée et/ou les signes cutanés du vieillissement, associé(e)(s) ou non à une sécheresse cutanée, chez un individu en ayant besoin, comprenant au moins les étapes consistant à :

a) effectuer, avant la mise en oeuvre du traitement cosmétique, dans un premier échantillon de peau isolé prélevé chez ledit individu, au moins une première mesure qualitative ou quantitative de l'expression, de la maturation ou de l'activité d'au moins une séquence d'acides aminés représentée par une séquence choisie parmi SEQ ID NO: 11, SEQ ID NO : 12, SEQ ID NO : 13, SEQ ID NO : 14, SEQ ID NO: 15, SEQ ID NO : 16, SEQ ID NO : 17, SEQ ID NO : 18, SEQ ID NO : 19, SEQ ID NO : 20, ou d'au moins une séquence d'acides nucléiques représentée par une séquence choisie parmi SEQ ID NO: 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10,
b) effectuer, après la mise en oeuvre du traitement cosmétique, dans un second échantillon de peau isolé prélevé chez ledit individu, au moins une seconde mesure, qualitative ou quantitative de l'expression, de la maturation ou de l'activité de ladite séquence d'acides aminés ou de ladite séquence d'acides nucléiques, et
c) comparer les première et deuxième mesures, notamment afin d'en déduire une information relative à un effet au moins de la mise en oeuvre du traitement cosmétique.

**11.** Procédé selon la revendication 10, dans lequel la séquence d'acides aminées est représentée parmi une séquence choisie parmi SEQ ID NO : 17 et SEQ ID NO : 18.

**12.** Peptide isolé représenté par la séquence d'acides aminés SEQ ID NO: 18.

**13.** Composition cosmétique comprenant un peptide tel que défini en revendication 12 ou une séquence d'acides nucléiques codant pour un tel peptide.

## Patentansprüche

**1.** Verwendung (i) von mindestens einer Aminosäuresequenz, die durch eine Nucleinsäuresequenz codiert wird, die durch eine Sequenz dargestellt ist, die ausgewählt ist aus SEQ ID NO:1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, oder (ii) der Nucleinsäuresequenz als Biomarker eines Zustands der gealterten Haut und/oder der Anzeichen von Hautalterung, die mit Hauttrockenheit einhergehen oder nicht.

**2.** Verwendung nach Anspruch 1, wobei die Aminosäuresequenz durch eine Sequenz dargestellt ist, die ausgewählt ist aus SEQ ID NO: 11 bis SEQ ID NO: 20, und vorzugsweise ausgewählt ist aus SEQ ID NO: 16, SEQ ID NO: 17, SEQ ID NO: 18, SEQ ID NO: 19, SEQ ID NO: 20.

**3.** Verwendung (i) von mindestens einer Aminosäuresequenz, die durch die eine Sequenz dargestellt ist, die ausgewählt ist aus SEQ ID NO: 11, SEQ ID NO: 12, SEQ ID NO: 13 und SEQ ID NO: 16 oder (ii) von mindestens einer Nucleinsäuresequenz, die durch eine Sequenz dargestellt ist, die ausgewählt ist aus SEQ ID NO: 1, SEQ ID NO: 2, SEQ ID NO: 3 und SEQ ID NO: 6, zum Screening auf Wirkstoffe oder äußerliche Behandlungen zur Pflege der gealterten Haut und/oder zur Vorbeugung und/oder Behandlung von Anzeichen von Hautalterung, die mit einer Hautaustrocknung einhergehen oder nicht, die gegenüber der Modulation der Aktivität, der Expression oder der Maturation der Aminosäuresequenz oder der Nucleinsäuresequenz empfindlich sind.

**4.** Verwendung (i) von mindestens einer Aminosäuresequenz, die durch eine Sequenz dargestellt ist, die ausgewählt ist aus SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, oder (ii) von mindestens einer Nucleinsäuresequenz, die durch eine Sequenz dargestellt ist, die ausgewählt ist aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, zur Charakterisierung der Wirksamkeit einer kosmetischen Behandlung der gealterten Haut und/oder der Anzeichen von Hautalterung, die mit Hauttrockenheit einhergehen oder nicht.

**5.** Kosmetische Verwendung einer wirksamen Menge (i) von mindestens einer Aminosäuresequenz, die durch eine Sequenz dargestellt ist, die ausgewählt ist aus SEQ ID NO: 11, SEQ ID NO: 12, SEQ ID NO: 13 und SEQ ID NO: 16, oder (ii) von mindestens einer Nucleinsäuresequenz, die durch eine Sequenz dargestellt ist, die ausgewählt ist aus SEQ ID NO: 1, SEQ ID NO: 2, SEQ ID NO: 3 und SEQ ID NO: 6, als Wirkstoff zur Vorbeugung und/oder Behandlung der gealterten Haut und/oder der Anzeichen von Hautalterung die mit Hauttrockenheit einhergehen oder nicht.

**6.** Verwendung einer wirksamen Menge (i) von mindestens einer Aminosäuresequenz, die dargestellt ist durch eine Sequenz, die ausgewählt ist aus SEQ ID NO: 11, SEQ ID NO: 12, SEQ ID NO: 13 und SEQ ID NO: 16 oder (ii) von mindestens einer Nucleinsäuresequenz, die dargestellt ist durch eine Sequenz, die ausgewählt ist aus SEQ ID NO: 1, SEQ ID NO: 2, SEQ ID NO: 3 und SEQ ID NO: 6, zur Herstellung eines pluristratifizierten epithelialen Zellmodells, vorzugsweise eines Modells von rekonstruierter Haut, das eine gealterte Haut nachbildet, gegebenenfalls in Verbindung mit Hautaustrocknung.

**7.** *In vivo* oder *ex vivo* Verfahren zur Charakterisierung eines Zustands der gealterten Haut und/oder der Anzeichen von Hautalterung, die mit Hauttrockenheit einhergehen oder nicht, umfassend mindestens die Schritte bestehend aus:

a) Durchführen, in einer isolierten Probe einer Haut, einer qualitativen oder quantitativen Messung der Expression, der Maturation oder der Aktivität von mindestens einer Aminosäuresequenz, die dargestellt ist durch eine Sequenz, die ausgewählt ist aus SEQ ID NO: 11, SEQ ID NO: 12, SEQ ID NO: 13, SEQ ID NO: 14, SEQ ID NO: 15, SEQ ID NO:16, SEQ ID NO: 17, SEQ ID NO: 18, SEQ ID NO: 19, SEQ ID NO: 20 oder von mindestens einer Nucleinsäuresequenz, die dargestellt ist durch eine Sequenz, die ausgewählt ist aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, und
b) Vergleichen der in Schritt a) durchgeführten Messung mit einer Referenzmessung.

**8.** Verfahren zum Screening *in vitro* oder *ex vivo* von Wirkstoffen oder äußerlichen Behandlungen zur Pflege der gealterten Haut und/oder zur Vorbeugung und/oder Behandlung von Anzeichen von Hautalterung, die mit Hauttrockenheit einhergehen oder nicht, die gegenüber der Modulation der Aktivität, der Expression oder der Maturation einer Aminosäuresequenz empfindlich sind, die durch eine Sequenz dargestellt ist, die ausgewählt aus SEQ ID NO: 11, SEQ ID NO: 12, SEQ ID NO: 13 und SEQ ID NO: 16 oder einer Nucleinsäuresequenz, die dargestellt ist durch eine Sequenz, die ausgewählt ist aus SEQ ID NO: 1, SEQ ID NO: 2, SEQ ID NO: 3 und SEQ ID NO: 6, umfassend mindestens die Schritte bestehend aus:

a) Unterziehen der Aminosäuresequenz oder der Nucleinsäuresequenz unter Bedingungen, die für die Aktivität, die Expression oder die Maturation der Sequenzen günstig sind,
b) In-Kontakt-Bringen der Aminosäuresequenz oder der Nucleinsäuresequenz mit mindestens einem zu testenden Wirkstoff, oder Exposition der Aminosäuresequenz oder der Nucleinsäuresequenz gegenüber einer zu testenden äußerlichen Behandlung,
c) Durchführen einer qualitativen oder quantitativen Messung der Expression, der Maturation oder der Aktivität der Aminosäuresequenz oder der Nucleinsäuresequenz, und
d) Vergleichen der Messung mit einer Referenzmessung, und
e) Feststellen einer Differenz zwischen der Messung und der Referenzmessung, um den Wirkstoff oder die Behandlung als geeignet zur Vorbeugung und/oder Behandlung der gealterten Haut und/oder der Hautalterungsanzeichen, die mit Hauttrockenheit einhergehen oder nicht, zu charakterisieren.

**9.** Kosmetisches Verfahren zur Vorbeugung und/oder Behandlung einer gealterten Haut und/oder der Hautalterungsanzeichen die mit Hauttrockenheit einhergehen oder nicht, bei einem Individuum, das Bedarf daran hat, umfassend mindestens einen Schritt bestehend aus der Verabreichung an das Individuum von mindestens einer Zusammensetzung, umfassend als Wirkstoff (i) mindestens eine Aminosäuresequenz, die durch eine Sequenz dargestellt ist, ausgewählt aus SEQ ID NO: 11, SEQ ID NO: 12 , SEQ ID NO: 13 und SEQ ID NO: 16 oder (ii) mindestens eine Nucleinsäuresequenz, die durch eine Sequenz dargestellt ist, die ausgewählt ist aus SEQ ID NO: 1, SEQ ID NO: 2, SEQ ID NO: 3 und SEQ ID NO: 6.

**10.** Kosmetisches Verfahren *in vitro* oder *ex vivo* zur Charakterisierung der Wirksamkeit einer kosmetischen Behandlung der gealterten Haut und/oder der Hautalterungsanzeichen, die mit Hauttrockenheit einhergehen oder nicht, bei einem Individuum, das Bedarf daran hat, umfassend mindestens die Schritte bestehend aus:

a) Durchführen vor der Durchführung der kosmetischen Behandlung in einer ersten Probe von isolierter, dem Individuum entnommener Haut von mindestens einer ersten qualitativen oder quantitativen Messung der Expression, der Maturation oder der Aktivität von mindestens einer Aminosäuresequenz, die durch eine Sequenz dargestellt ist, die ausgewählt ist aus SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, oder von mindestens einer Nucleinsäuresequenz, die durch eine Sequenz dargestellt ist, die ausgewählt ist aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10,
b) Durchführen, nach der Durchführung der kosmetischen Behandlung in einer zweiten Probe von dem Individuum entnommener isolierter Haut von mindestens einer zweiten qualitativen oder quantitativen Messung der Expression, der Maturation oder der Aktivität der Aminosäuresequenz oder der Nucleinsäuresequenz, und
c) Vergleichen der ersten und der zweiten Messung, insbesondere, um daraus eine relative Information über eine Wirkung mindestens der Durchführung der kosmetischen Behandlung abzuleiten.

**11.** Verfahren nach Anspruch 10, wobei die Aminosäuresequenz durch eine Sequenz dargestellt ist, die ausgewählt ist aus SEQ ID NO: 17 und SEQ ID NO: 18.

**12.** Isoliertes Peptid, das durch die Aminosäuresequenz SEQ ID NO: 18 dargestellt ist.

**13.** Kosmetische Zusammensetzung, umfassend ein Peptid, wie in Anspruch 12 definiert, oder eine Nucleinsäuresequenz, die ein solches Peptid codiert.

## Claims

**1.** The use (i) of at least one sequence of amino-acids coded by a sequence of nucleic acids represented by a sequence selected from among SEQ ID NO: 1, SEQ ID NO: 2 SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 or (ii) of said sequence of nucleic acids, as a biomarker of an aged skin condition and/or skin signs of ageing, either associated or not with skin dryness.

**2.** The use according to claim 1, wherein said sequence of amino-acids is represented by a sequence selected from among SEQ ID NO: 11 to SEQ ID NO: 20, and is preferably selected from among SEQ ID NO: 16, SEQ ID NO: 17, SEQ ID NO: 18, SEQ ID NO: 19, SEQ ID NO: 20.

**3.** The use (i) of at least one sequence of amino-acids represented by a sequence selected from among SEQ ID NO: 11, SEQ ID NO: 12, SEQ ID NO: 13 and SEQ ID NO: 16, or (ii) of at least one sequence of nucleic acids represented by a sequence selected from among SEQ ID NO: 1, SEQ ID NO: 2 SEQ ID NO: 3, and SEQ ID NO: 6, for screening active agents or physical treatments for the care of aged skin and/or preventing and/or treating skin signs of ageing either associated or not with skin dryness, which may modulate the activity, the expression or the maturation of said sequence of amino-acids or of said sequence of nucleic acids.

**4.** The use (i) of at least one sequence of amino-acids represented by a sequence selected from among SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, or (ii) of at least one sequence of nucleic acids represented by a sequence selected from among SEQ ID NO: 1, SEQ ID NO: 2 SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 for characterizing the effectiveness of a cosmetic treatment of aged skin, and/or skin signs of ageing, either associated or not with skin dryness.

**5.** The cosmetic use of an effective amount (i) of at least one sequence of amino-acids represented by a sequence selected from among SEQ ID NO: 11, SEQ ID NO: 12, SEQ ID NO: 13 and SEQ ID NO: 16, or (ii) of at least one sequence of nucleic acids represented by a sequence selected from among SEQ ID NO: 1, SEQ ID NO: 2 SEQ ID NO: 3, and SEQ ID NO: 6, as an active agent for preventing and/or treating aged skin and/or skin signs of ageing either associated or not with skin dryness.

**6.** The use of an effective amount (i) of at least one sequence of amino-acids represented by a sequence selected from among SEQ ID NO: 11, SEQ ID NO: 12, SEQ ID NO: 13 and SEQ ID NO: 16, or (ii) of at least one sequence of nucleic acids represented by a sequence selected from among SEQ ID NO: 1, SEQ ID NO: 2 SEQ ID NO: 3, and SEQ ID NO: 6, for preparing a pluristratified epithelial cell model, preferably a rebuilt skin model, mimicking an aged skin, optionally associated with skin dryness.

**7.** An *in vitro* or *ex vivo* method for characterizing an aged skin condition and/or skin signs of ageing either associated or not with skin dryness, comprising at least the steps consisting of:

a) conducting, in an isolated sample of a skin, a qualitative or quantitative measurement of the expression, the maturation, or the activity of at least one sequence of amino-acids represented by a sequence selected from among SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, or of at least one sequence of nucleic acids represented by a sequence selected from among SEQ ID NO: 1, SEQ ID NO: 2 SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, and
b) comparing said measurement conducted in step a) with a reference measurement.

**8.** A method for screening *in vitro* or *ex vivo* active agents or physical treatments for the care of aged skin and/or preventing and/or treating skin signs of ageing either associated or not with skin dryness, which may modulate the activity, the expression or the maturation of a sequence of amino-acids represented by a sequence selected from among SEQ ID NO: 11, SEQ ID NO: 12, SEQ ID NO: 13 and SEQ ID NO: 16, or of a sequence of nucleic acids represented by a sequence selected from among SEQ ID NO: 1, SEQ ID NO: 2 SEQ ID NO: 3, and SEQ ID NO: 6, comprising at least the steps consisting of:

a) disposing of said sequence of amino-acids or said sequence of nucleic acids under favorable conditions for the activity, the expression or the maturation of said sequences,
b) putting said sequence of amino-acids or said sequence of nucleic acids in contact with an active agent to be tested or exposing said sequence of amino-acids or said sequence of nucleic acids with a physical treatment to be tested,
c) conducting a qualitative or quantitative measurement of the expression, the maturation, or of the activity of said sequence of amino-acids or said sequence of nucleic acids, and
d) comparing said measurement with a reference measurement, and
e) observing a difference between said measurement and the reference measurement for characterizing the active agent or the treatment as being useful for preventing and/or treating aged skin and/or signs of aged skin, either associated or not with skin dryness.

**9.** A cosmetic method for preventing and/or treating aged skin and/or signs of aged skin, either associated or not with skin dryness in an individual in need thereof, comprising at least one step consisting of administering to said individual, at least one composition comprising as an active agent (i) at least one sequence of amino-acids represented by a sequence selected from among SEQ ID NO: 11, SEQ ID NO: 12, SEQ ID NO: 13 and SEQ ID NO: 16, or (ii) at least one sequence of nucleic acids represented by a sequence selected from among SEQ ID NO: 1, SEQ ID NO: 2 SEQ ID NO: 3, and SEQ ID NO: 6.

**10.** A cosmetic method, *in vitro* or *ex vivo*, for characterizing the effectiveness of a cosmetic treatment of aged skin and/or skin signs of ageing associated or not with skin dryness, in an individual in need thereof, comprising at least the steps consisting of:

a) conducting, before applying the cosmetic treatment, in a first isolated skin sample taken from said individual, at least one first qualitative or quantitative measurement of the expression, the maturation or of the activity of at least one sequence of amino-acids represented by a sequence selected from among SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, or at least one sequence of nucleic acids represented by a sequence selected from among SEQ ID NO: 1, SEQ ID NO: 2 SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10,
b) conducting, after applying the cosmetic treatment, in a second isolated skin sample taken from said individual, at least one second qualitative or quantitative measurement of the expression, the maturation or of the activity of said sequence of amino-acids or of said sequence of nucleic acids, and
c) comparing the first and second measurements, notably in order to infer therefrom a piece of information relative to at least one effect of the application of the cosmetic treatment.

**11.** The method according to claim 10, wherein the sequence of amino-acids is represented by a sequence selected from among SEQ ID NO: 17 and SEQ ID NO: 18.

12. An isolated peptide, represented by the sequence of amino-acids SEQ ID NO: 18.

13. A cosmetic composition comprising a peptide as defined in claim 12, or a sequence of nucleic acids coding for such a peptide.

# Listage de Séquences

**SEQ ID NO: 1** (P81605)

```
atgaggttca tgactctcct cttcctgaca gctctggcag gagccctggt ctgtgcctat      60
gatccagagg ccgcctctgc cccaggatcg gggaaccctt gccatgaagc atcagcagct     120
caaaaggaaa atgcaggtga agacccaggg ttagccagac aggcaccaaa gccaaggaag     180
cagagatcca gccttctgga aaaaggccta gacggagcaa aaaaagctgt gggggactc     240
ggaaaactag gaaaagatgc agtcgaagat ctagaaagcg tgggtaaagg agccgtccat     300
gacgttaaag acgtccttga ctcagtacta tag                                   333
```

**SEQ ID NO: 2** (A5JHP2)

```
atgaggttca tgactctcct cttcctgaca gctctggcag gagccctggt ctgtgcctat      60
gatccagagg ccgcctctgc cccaggatcg gggaaccata aacaaatgga ttgtttacag     120
ctacagaagc ccccttcaga gactgccaaa tttctgtcct catccaccaa cctgcctaga     180
agagagaagc tagtgccctc tgcaaaacct ccccacacta gggggctggt ataa            234
```

**SEQ ID NO: 3** (A5JHP3)

```
atgaggttca tgactctcct cttcctgaca gctctggcag gagccctggt ctgtgcctat      60
gatccagagg ccgcctctgc cccaggatcg gggaaccctt gccatgaagc atcagcagct     120
caaaaggaaa atgcaggtga agacccaggg ttagccagac aggcaccaaa gccaaggaag     180
cagagatcca gccttctgga aaaaggccta gacggagcaa aaaaagctgt gggggactc     240
ggaaaactag gaaaagatgc agtcgaagat ctagaaagcg tgggtaaagg tggggaagag     300
aggttggtct ttggggctcc tgtgaatcta acctccatcc ctctgacttc tgtgagccgt     360
ccatga                                                                 366
```

**SEQ ID NO: 4** (signal peptide P81605)

```
atgaggttca tgactctcct cttcctgaca gctctggcag gagccctggt ctgtgcc          57
```

**SEQ ID NO: 5** (survival peptide P81605)

```
tatgatccag aggccgcctc tgccccagga tcggggaacc cttgccatga agcatcagca      60
gctcaaaagg aaaatgcagg tgaagaccca                                       90
```

**SEQ ID NO: 6** (Dermcidine)

```
tatgatccag aggccgcctc tgccccagga tcggggaacc cttgccatga agcatcagca 60
gctcaaaagg aaaatgcagg tgaagaccca gggttagcca gacaggcacc aaagccaagg   120
aagcagagat ccagccttct ggaaaaaggc ctagacggag caaaaaaagc tgtgggggga   180
ctcggaaaac taggaaaaga tgcagtcgaa gatctagaaa gcgtgggtaa aggagccgtc   240
catgacgtta aagacgtcct tgactcagta ctatag                             276
```

**SEQ ID NO: 7** (peptide 1)

```
gaaaatgcag gtgaagaccc agggttagcc aga                                    33
```

**SEQ ID NO: 8** (peptide 2)

```
aaagctgtgg ggggactcgg aaaactagga aaagatgcag tcgaagatct agaaagcgtg      60
ggtaaaggag ccgtccatga cgttaaa                                           87
```

**SEQ ID NO: 9**

```
agccttctgg aaaaaggcct agacggagca aaaaaagctg tgggggggact cggaaaacta      60
ggaaaagatg cagtcgaaga tctagaaagc gtgggtaaag gagccgtcca tgacgttaaa     120
gacgtccttg actcagta                                                   138
```

**SEQ ID NO: 10**

```
agccttctgg aaaaaggcct agacggagca aaaaaagctg tgggggggact cggaaaacta      60
ggaaaagatg cagtcgaaga tctagaaagc gtgggtaaag gagccgtcca tgacgttaaa     120
gacgtccttg actcagtact a                                               141
```

**SEQ ID NO: 11**(P81605)

```
        10         20         30         40         50         60
MRFMTLLFLT ALAGALVCAY DPEAASAPGS GNPCHEASAA QKENAGEDPG LARQAPKPRK

        70         80         90        100        110
QRSSLLEKGL DGAKKAVGGL GKLGKDAVED LESVGKGAVH DVKDVLDSVL
```

**SEQ ID NO: 12** (A5JHP2)

```
        10         20         30         40         50         60
MRFMTLLFLT ALAGALVCAY DPEAASAPGS GNHKQMDCLQ LQKPPSETAK FLSSSTNLPR

        70
```

```
REKLVPSAKP PHTRGLV
```

**SEQ ID NO: 13** (A5JHP3)

```
        10         20         30         40         50         60
MRFMTLLFLT ALAGALVCAY DPEAASAPGS GNPCHEASAA QKENAGEDPG LARQAPKPRK

        70         80         90        100        110        120
QRSSLLEKGL DGAKKAVGGL GKLGKDAVED LESVGKGGEE RLVFGAPVNL TSIPLTSVSR P
```

**SEQ ID NO: 14** (signal)

```
        10
MRFMTLLFLT ALAGALVCA
```

**SEQ ID NO: 15** (survival peptide)

```
        10         20         30
YDPEAASAPG SGNPCHEASA AQKENAGEDP
```

**SEQ ID NO: 16** (Dermcidine)

```
        10         20         30         40         50         60
YDPEAASAPG SGNPCHEASA AQKENAGEDP GLARQAPKPR KQRSSLLEKG LDGAKKAVGG

        70         80         90
LGKLGKDAVE DLESVGKGAV HDVKDVLDSV L
```

**SEQ ID NO: 17** (peptide 1)

```
        10
ENAGEDPGLA R
```

**SEQ ID NO: 18** (peptide 2)

```
        10         20
KAVGGLGKLG KDAVEDLESV GKGAVHDVK
```

**SEQ ID NO: 19** (Dermcidine) DCD-1

```
         10         20         30         40
SSLLEKGLDG AKKAVGGLGK LGKDAVEDLE SVGKGAVHDV KDVLDSV
```

**<u>SEQ ID NO: 20</u>** (Dermcidine) DCD-1L

```
         10         20         30         40
SSLLEKGLDG AKKAVGGLGK LGKDAVEDLE SVGKGAVHDV KDVLDSVL
```

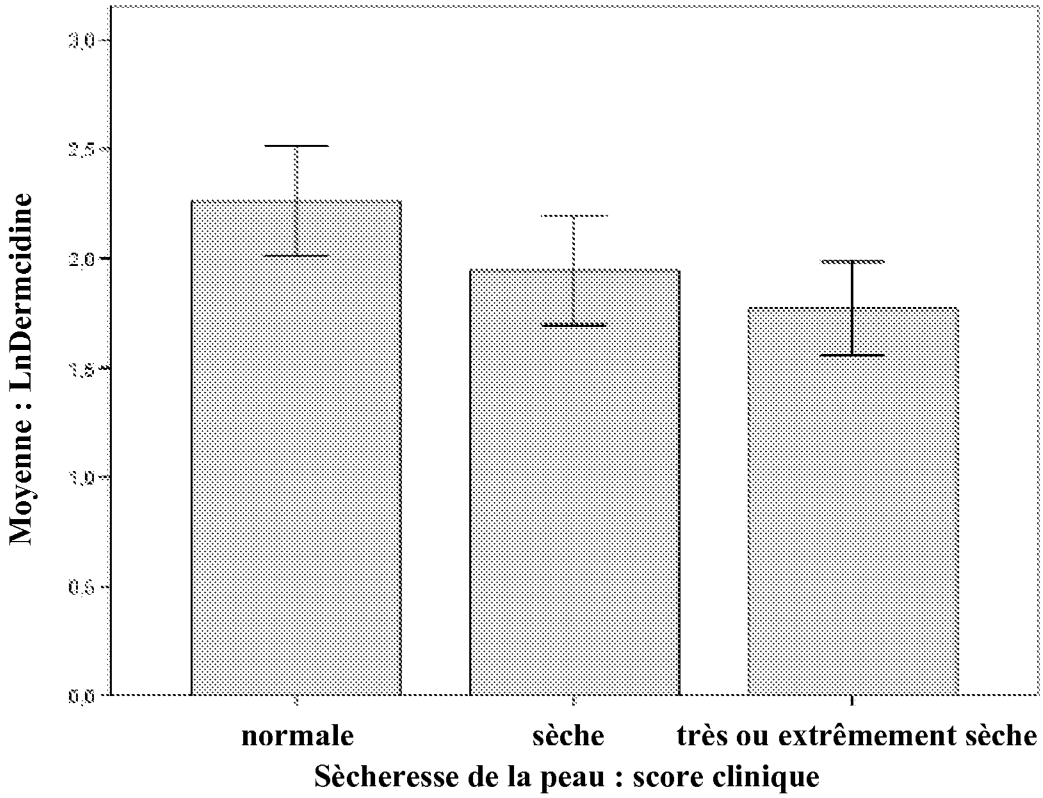
3,0
2,5
2,0
1,5
1,0
0,5
0,0
Moyenne : LnDermcidine
normale
sèche
très ou extrêmement sèche
Sècheresse de la peau : score clinique

FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2924946
- US 5834192 A
- US 2008020976 A
- FR 2667778
- FR 1057690
- FR 1057699
- US 61344826 B
- US 61344827 B


### Littérature non-brevet citée dans la description


- **KYTE et al.** *J. Mol. Biol.,* 1982, vol. 157, 105
- **SAMBROOK et al.** Molecular Cloning - A Laboratory Manual. Coldspring Harbor Laboratory, 1989, vol. I-III
- Antibodies: A Laboratory Manual. Cold Spring Harbor Laboratory Press, 1990
- **ZIESKE.** *J. Exp. Bot.,* 2006, vol. 57, 1501
- **WIESE et al.** *Proteomics,* 2007, vol. 7, 340
- **MEHUL et al.** *J. Biol. Chem.,* 2000, vol. 275 (17), 12841-7
- **ANDERSON, R. K. ; W. L. KENNEY.** Effect of age on heat-activated sweat gland density and flow during exercise in dry heat. *J. Appl. Physiol.,* 1987, vol. 63 (3), 1089-1094
- **BAECHLE, D. ; T. FLAD et al.** Cathepsin D is present in human eccrine sweat and involved in the postsecretory processing of the antimicrobial peptide DCD-1L. *J. Biol. Chem.,* 2006, vol. 281 (9), 5406-5415
- **CUNNINGHAM, T. J. ; L. HODGE et al.** Identification of a survival-promoting peptide in medium conditioned by oxidatively stressed cell lines of nervous system origin. *J. Neurosci,* 1998, vol. 18 (18), 7047-7060
- **FLAD, T. ; R. BOGUMIL et al.** Detection of dermcidin-derived peptides in sweat by ProteinChip Technology. *J. Immunol. Methods,* 2002, vol. 270 (1), 53-62
- **KENNEY, W. L. ; S. R. FOWLER.** Methylcholine-activated eccrine sweat gland density and output as a function of age. *J. Appl. Physiol.,* 1988, vol. 65 (3), 1082-1086
- **MEHUL B. et al.** Identification and Cloning of a New Calmodulin-like Protein from Human Epidermi. *J. Biol. Chem.,* 2000, vol. 275 (17), 12841-12847
- **MOREIRA, D. F ; B. E. STRAUSS et al.** Genes up- and down-regulated by dermcidin in breast cancer: a microarray analysis. *Genet. Mol. Res.,* 2008, vol. 7 (3), 925-932
- **NIYONSABA, F. A. SUZUKI et al.** The human antimicrobial peptide dermcidin activates normal human keratinocytes. *Br. J. Dermatol.,* 2009, vol. 160 (2), 243-249
- **RIEG, S.C., GARBE et al.** Dermcidin is constitutively produced by eccrine sweat gland and is not induced in epidermal cells under inflammatory skin conditions. *Br. J. Dermatol.,* 2004, vol. 151 (3), 534-539
- **RIEG S., II STEFFEN et al.** Deficiency of dermcidin-derived antimicrobial peptides in sweat of patients with atopic dermatitis correlates with an impaired innate defense of human skin in vivo. *J. Immunol,* 2005, vol. 174 (12), 8003-8010
- **SAKURADA K. ; T. AKUTSU et al.** Detection of dermcidin for sweat identification by real-time RT-PCR and ELISA. *Forensic. Sci. Int.,* 13 Novembre 2009, vol. 194 (1-3), 80-4
- **SAMBROOK et al.** Coldspring Harbor Laboratory. Coldspring Harbor Press, 1989, vol. I-III
- **SCHITTEK, B ; R. HIPFEL et al.** Dermcidin: a novel human antibiotic peptide secreted by sweat glands. *Nat. Immunol,* 2001, vol. 2 (12), 1133-11137
- **WATCHORN, T. M. ; N. DOWIDAR et al.** The chachectic mediator proteolysis inducing factor activates NF-kappaB and STAT3 in human Kupffer cells and monocytes. *Int. J. Oncol.,* 2005, vol. 27 (4), 1105-1111
- **WIESE et al.** Protein labeling by iTRAQ: A new tool for quantitative mass spectrometry in proteome research. *Proteomics,* 2007, vol. 7 (3), 340-350
- **ZIESKE et al.** A perspective on the use of iTRAQ reagent technology for protein complex and profiling studies. *J. Exp. Bot.,* 2006, vol. 57 (7), 1051-1058

SEQUENCE LISTING

<110> L'OREAL

<120> Utilisation cosmétique de la Dermcidine, analogues ou fragments de celle-ci

<130> BR94152/KLP/SLH/cf

<150> FR 10 57690
<151> 2010-09-24

<150> FR 10 57699
<151> 2010-09-24

<150> US 61/344826
<151> 2010-10-19

<150> US 61/344827
<151> 2010-10-19

<160> 20

<170> PatentIn version 3.3

<210> 1
<211> 333
<212> DNA
<213> Homo Sapiens

<400> 1

```
atgaggttca tgactctcct cttcctgaca gctctggcag gagccctggt ctgtgcctat      60
gatccagagg ccgcctctgc cccaggatcg gggaaccctt gccatgaagc atcagcagct     120
caaaaggaaa atgcaggtga agacccaggg ttagccagac aggcaccaaa gccaaggaag     180
cagagatcca gccttctgga aaaaggccta gacggagcaa aaaaagctgt gggggggactc    240
ggaaaactag gaaaagatgc agtcgaagat ctagaaagcg tgggtaaagg agccgtccat     300
gacgttaaag acgtccttga ctcagtacta tag                                  333
```

<210> 2
<211> 234
<212> DNA
<213> Homo Sapiens

<400> 2

```
atgaggttca tgactctcct cttcctgaca gctctggcag gagccctggt ctgtgcctat      60
gatccagagg ccgcctctgc cccaggatcg gggaaccata aacaaatgga ttgtttacag     120
ctacagaagc cccctcaga gactgccaaa tttctgtcct catccaccaa cctgcctaga      180
agagagaagc tagtgccctc tgcaaaacct ccccacacta gggggctggt ataa           234
```

<210> 3
<211> 366
<212> DNA
<213> Homo Sapiens

<400> 3

```
atgaggttca tgactctcct cttcctgaca gctctggcag gagccctggt ctgtgcctat      60
gatccagagg ccgcctctgc cccaggatcg gggaaccctt gccatgaagc atcagcagct     120
caaaaggaaa atgcaggtga agacccaggg ttagccagac aggcaccaaa gccaaggaag     180
cagagatcca gccttctgga aaaaggccta gacggagcaa aaaaagctgt gggggactc      240
ggaaaactag gaaaagatgc agtcgaagat ctagaaagcg tgggtaaagg tggggaagag     300
aggttggtct ttggggctcc tgtgaatcta acctccatcc ctctgacttc tgtgagccgt     360
ccatga                                                                366
```

<210> 4
<211> 57
<212> DNA
<213> Homo Sapiens

<400> 4
atgaggttca tgactctcct cttcctgaca gctctggcag gagccctggt ctgtgcc 57

<210> 5
<211> 90
<212> DNA
<213> Homo Sapiens

<400> 5

```
tatgatccag aggccgcctc tgccccagga tcggggaacc cttgccatga agcatcagca      60
gctcaaaagg aaaatgcagg tgaagaccca                                       90
```

<210> 6
<211> 276
<212> DNA
<213> Homo Sapiens

<400> 6

tatgatccag aggccgcctc tgccccagga tcggggaacc cttgccatga agcatcagca 60

gctcaaaagg aaaatgcagg tgaagaccca gggttagcca gacaggcacc aaagccaagg 120

aagcagagat ccagccttct ggaaaaaggc ctagacggag caaaaaaagc tgtgggggga 180

ctcggaaaac taggaaaaga tgcagtcgaa gatctagaaa gcgtgggtaa aggagccgtc 240

catgacgtta aagacgtcct tgactcagta ctatag 276

<210> 7
<211> 33
<212> DNA
<213> Homo Sapiens

<400> 7
gaaaatgcag gtgaagaccc agggttagcc aga 33

<210> 8
<211> 87
<212> DNA
<213> Homo Sapiens

<400> 8

aaagctgtgg ggggactcgg aaaactagga aaagatgcag tcgaagatct agaaagcgtg 60

ggtaaaggag ccgtccatga cgttaaa 87

<210> 9
<211> 138
<212> DNA
<213> Homo Sapiens

<400> 9

agccttctgg aaaaaggcct agacggagca aaaaaagctg tggggggact cggaaaacta 60

ggaaaagatg cagtcgaaga tctagaaagc gtgggtaaag gagccgtcca tgacgttaaa 120

gacgtccttg actcagta 138

<210> 10
<211> 141
<212> DNA
<213> Homo Sapiens

<400> 10

agccttctgg aaaaaggcct agacggagca aaaaaagctg tggggggact cggaaaacta 60

ggaaaagatg cagtcgaaga tctagaaagc gtgggtaaag gagccgtcca tgacgttaaa 120

gacgtccttg actcagtact a 141

<210> 11

<211> 110
<212> PRT
<213> Homo Sapiens

<400> 11

```
Met Arg Phe Met Thr Leu Leu Phe Leu Thr Ala Leu Ala Gly Ala Leu
1               5                   10                  15

Val Cys Ala Tyr Asp Pro Glu Ala Ala Ser Ala Pro Gly Ser Gly Asn
            20                  25                  30

Pro Cys His Glu Ala Ser Ala Ala Gln Lys Glu Asn Ala Gly Glu Asp
        35                  40                  45

Pro Gly Leu Ala Arg Gln Ala Pro Lys Pro Arg Lys Gln Arg Ser Ser
    50                  55                  60

Leu Leu Glu Lys Gly Leu Asp Gly Ala Lys Lys Ala Val Gly Gly Leu
65                  70                  75                  80

Gly Lys Leu Gly Lys Asp Ala Val Glu Asp Leu Glu Ser Val Gly Lys
                85                  90                  95

Gly Ala Val His Asp Val Lys Asp Val Leu Asp Ser Val Leu


          100                 105                 110
```

<210> 12
<211> 77
<212> PRT
<213> Homo Sapiens

<400> 12

```
Met Arg Phe Met Thr Leu Leu Phe Leu Thr Ala Leu Ala Gly Ala Leu
1               5                   10                  15

Val Cys Ala Tyr Asp Pro Glu Ala Ala Ser Ala Pro Gly Ser Gly Asn
            20                  25                  30

His Lys Gln Met Asp Cys Leu Gln Leu Gln Lys Pro Pro Ser Glu Thr
        35                  40                  45

Ala Lys Phe Leu Ser Ser Ser Thr Asn Leu Pro Arg Arg Glu Lys Leu
    50                  55                  60

Val Pro Ser Ala Lys Pro Pro His Thr Arg Gly Leu Val
65                  70                  75
```

<210> 13
<211> 121
<212> PRT
<213> Homo Sapiens

<400> 13

```
Met Arg Phe Met Thr Leu Leu Phe Leu Thr Ala Leu Ala Gly Ala Leu
1               5                   10                  15

Val Cys Ala Tyr Asp Pro Glu Ala Ala Ser Ala Pro Gly Ser Gly Asn
            20                  25                  30

Pro Cys His Glu Ala Ser Ala Ala Gln Lys Glu Asn Ala Gly Glu Asp
        35                  40                  45

Pro Gly Leu Ala Arg Gln Ala Pro Lys Pro Arg Lys Gln Arg Ser Ser
    50                  55                  60

Leu Leu Glu Lys Gly Leu Asp Gly Ala Lys Lys Ala Val Gly Gly Leu
65                  70                  75                  80

Gly Lys Leu Gly Lys Asp Ala Val Glu Asp Leu Glu Ser Val Gly Lys
                85                  90                  95

Gly Gly Glu Glu Arg Leu Val Phe Gly Ala Pro Val Asn Leu Thr Ser
            100                 105                 110

Ile Pro Leu Thr Ser Val Ser Arg Pro
        115                 120
```

<210> 14
<211> 19
<212> PRT
<213> Homo Sapiens

<400> 14

```
Met Arg Phe Met Thr Leu Leu Phe Leu Thr Ala Leu Ala Gly Ala Leu
1               5                   10                  15

Val Cys Ala
```

<210> 15
<211> 30
<212> PRT
<213> Homo Sapiens

<400> 15

```
Tyr Asp Pro Glu Ala Ala Ser Ala Pro Gly Ser Gly Asn Pro Cys His
1               5                   10                  15

Glu Ala Ser Ala Ala Gln Lys Glu Asn Ala Gly Glu Asp Pro
            20                  25                  30
```

<210> 16
<211> 91
<212> PRT
<213> Homo Sapiens

<400> 16

```
Tyr Asp Pro Glu Ala Ala Ser Ala Pro Gly Ser Gly Asn Pro Cys His
1               5                   10                  15

Glu Ala Ser Ala Ala Gln Lys Glu Asn Ala Gly Glu Asp Pro Gly Leu
            20                  25                  30

Ala Arg Gln Ala Pro Lys Pro Arg Lys Gln Arg Ser Ser Leu Leu Glu
        35                  40                  45

Lys Gly Leu Asp Gly Ala Lys Lys Ala Val Gly Gly Leu Gly Lys Leu
    50                  55                  60

Gly Lys Asp Ala Val Glu Asp Leu Glu Ser Val Gly Lys Gly Ala Val
65                  70                  75                  80

His Asp Val Lys Asp Val Leu Asp Ser Val Leu
                85                  90
```

<210> 17
<211> 11
<212> PRT
<213> Homo Sapiens

<400> 17

```
Glu Asn Ala Gly Glu Asp Pro Gly Leu Ala Arg
1               5                   10
```

<210> 18
<211> 29
<212> PRT
<213> Homo Sapiens

<400> 18

```
Lys Ala Val Gly Gly Leu Gly Lys Leu Gly Lys Asp Ala Val Glu Asp
1               5                   10                  15

Leu Glu Ser Val Gly Lys Gly Ala Val His Asp Val Lys
            20                  25
```

<210> 19
<211> 47
<212> PRT
<213> Homo Sapiens

<400> 19

```
Ser Ser Leu Leu Glu Lys Gly Leu Asp Gly Ala Lys Lys Ala Val Gly
1               5                   10                  15

Gly Leu Gly Lys Leu Gly Lys Asp Ala Val Glu Asp Leu Glu Ser Val
            20                  25                  30

Gly Lys Gly Ala Val His Asp Val Lys Asp Val Leu Asp Ser Val
        35                  40                  45
```

<210> 20
<211> 48
<212> PRT
<213> Homo Sapiens

<400> 20

```
Ser Ser Leu Leu Glu Lys Gly Leu Asp Gly Ala Lys Lys Ala Val Gly
1               5                   10                  15

Gly Leu Gly Lys Leu Gly Lys Asp Ala Val Glu Asp Leu Glu Ser Val
            20                  25                  30

Gly Lys Gly Ala Val His Asp Val Lys Asp Val Leu Asp Ser Val Leu
        35                  40                  45
```